(19) Europäisches Patentamt
European Patent Office
Office européen des brevets

(11) EP 1 426 767 A1

(12) EUROPEAN PATENT APPLICATION

(43) Date of publication:
**09.06.2004 Bulletin 2004/24**

(51) Int Cl.[7]: **G01N 33/68**, C12Q 1/44, C12Q 1/68

(21) Application number: **02026971.8**

(22) Date of filing: **04.12.2002**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(71) Applicant: **Bayer HealthCare AG**
**51368 Leverkusen (DE)**

(72) Inventors:
- **Münter, Klaus**
  **68163 Mannheim (DE)**
- **Bischoff, Erwin**
  **42115 Wuppertal (DE)**
- **Ellinghaus, Peter**
  **42113 Wuppertal (DE)**
- **Golz, Stefan**
  **45329 Essen (DE)**
- **Brüggemeier, Ulf**
  **42799 Leichlingen (DE)**
- **Geerts, Andreas**
  **42113 Wuppertal (DE)**

(54) **Diagnostics and therapeutics for diseases associated with human phosphodiesterase 4c (PDE4C)**

(57) The invention provides a human PDE4C which is associated with the disorders of the peripheral and central nervous system, cardiovascular diseases (in particular angina and coronary heart disease), hematological diseases, inflammation, cancer, gastroenterological diseases and respiratory disease. The invention also provides assays for the identification of compounds useful in the treatment or prevention of disorders of the peripheral and central nervous system, cardiovascular diseases (in particular angina and coronary heart disease), hematological diseases, inflammation, cancer, gastroenterological diseases and respiratory disease. The invention also features compounds which bind to and/or activate or inhibit the activity of PDE4C as well as pharmaceutical compositions comprising such compounds.

EP 1 426 767 A1

## Description

### Technical field of the invention

The present invention is in the field of molecular biology, more particularly, the present invention relates to nucleic acid sequences and amino acid sequences of a human PDE4C and its regulation for the treatment of disorders of the peripheral and central nervous system, cardiovascular diseases, hematological diseases, inflammation, cancer, gastroenterological diseases and respiratory disease in mammals. In particular, inhibition of human PDE4C for the treatment of angina or coronary heart disease is disclosed.

### Background of the invention

PDE4C is a member of the enzyme family of phosphodiesterases (PDEs) [[Perry MJ et al., 1997], [Shelton et al., 1997], US 5686286, US 5672509, WO9620281]. PDEs catalyze the hydrolyzation of 3', 5' cyclic nucleotides. That results in the formation of the respective nucleoside 5' monophosphates. The cyclic nucleotides cAMP and cGMP serve as crucial second messengers in a number of cellular signaling pathways. The PDEs as well as the guanylyl and adenylyl cyclases, which synthesize the cyclic nucleotides, are important cellular components to regulate the concentration of cyclic nucleotides and, thus, to regulate the signal transduction pathways. Because of their central role in regulating second messenger levels PDEs have been considered chemotherapeutic targets and have been worked on extensively.

Several families of PDEs have been identified. The nomenclature system includes first a number that indicates the PDE family. To date, eleven families (PDE 1-11) are known which are classified by: (i) primary structure; (ii) substrate preference; (iii) response to different modulators; (iv) sensitivity to specific inhibitors; and (v) modes of regulation [Loughney and Ferguson, (1996)]. The number indicating the family is followed by a capital letter, indicating a distinct gene, and the capital letter followed by a second number, indicating a specific splice variant or a specific transcript that utilizes a unique transcription initiation site.

PDEs show of the following structural features:

All mammalian PDEs identified to date possess a highly conserved region of 270-300 amino acids in the carboxy terminal half of the protein [Charbonneau, et al. (1986)]. Here, the catalytic site for cAMP and/or cGMP hydrolysis and two putative zinc binding sites as well as family specific determinants are located [Beavo, (1995); Francis, et al. (1994)]. The amino terminal regions of the various PDEs are highly variable and include other family specific determinants and diverse regulatory motifs such as: (i) calmodulin binding sites (PDE1); (ii) non-catalytic cyclic GMP binding sites (PDE2, PDE5, PDE6); (iii) membrane targeting sites (PDE4); (iv) hydrophobic membrane association sites (PDE3); and (v) phosphorylation sites for either the calmodulin-dependent kinase II (PDE1), the cAMP-dependent kinase (PDE1, PDE3, PDE4), or the cGMP dependent kinase (PDE5) [Beavo, (1995); Manganiello, et al. (1995); Conti, et al. (1995)].

Members of the PDE1 family are calcium-calmodulin dependent. The group is composed of at least three genes with several splicing variants [Kakkar, R. et al. (1999)]; PDE1A and PDE1B preferentially hydrolyze cGMP while PDE1C is dualspecific, it exhibits a high affinity for both cAMP and cGMP. In vitro experiments show regulation of some PDE1 species by phosphorylation, which decreases the affinity of the enzyme for calmodulin [Kakkar, (1999)]. PDE1s have been shown to be expressed in lung, heart and brain.

The PDE2 family is characterized as being specifically stimulated by cGMP [Loughney and Ferguson, supra]. PDE2 species have been found in cerebellum, neocortex, heart, kidney, lung, pulmonary artery, and skeletal muscle [Sadhu, K. et al. (1999)]. Only one gene has been identified, PDE2A. The respective PDE2A protein is specifically inhibited by erythro-9-(2-hydroxy-3-nonyl)adenine (EHNA).

Two genes have been identified in the PDE3 family, PDE3A and PDE3B, both having high affinity for both cAMP and cGMP, although the $V_{max}$ for cGMP hydrolysis is low enough that cGMP functions as a competitive inhibitor for cAMP hydrolysis. Enzymes in the PDE3 family are specifically inhibited by cGMP. PDE3 enzymes are specifically inhibited by milrinone and enoximone [Loughney and Ferguson, supra].

PDE4s are specific for cAMP hydrolysis. The family is comprised of four genes, PDE4A, PDE4B, PDE4C, and PDE4D. The genes give rise to multiple splice variants and are expressed in airway smooth muscle, the vascular endothelium, and all inflammatory cells. The enzymes can be activated by cAMP-dependent phosphorylation. Members of this family are specifically inhibited by the anti-depressant drug rolipram.

PDE5 is highly selective for cGMP [Turko, I.V. et al. (1998)]. Members of PDE5 family bind cGMP at non-catalytic sites [McAllister-Lucas, L.M. (1995)]. CGMP binding at non-catalytic sides has been suggested to be important for phosphorylation by cGMP-dependent protein kinase. PDE5 is highly expressed in vascular smooth muscle, platelets, lung, and kidney. Only one gene, PDE5A, has been identified.

PDE6s, the photoreceptor enzymes specifically hydrolyze cGMP [Loughney and Ferguson, supra]. PDE6s possess 2 regulatory high affinity cGMP binding sides. Genes include PDE6A and PDE6B (the protein products of which dimerize and bind two copies of a smaller y inhibitory subunit to form rod PDE), in addition to PDE6C which associates with three smaller proteins to form cone PDE.

The PDE7 family effects cAMP hydrolysis but, in contrast to the PDE4 family, is not inhibited by rolipram [Loughney and Ferguson, supra]. Only one gene, PDE7A, has been identified. PDE7A gives rise to multiple splice variants. PDE7 mRNA can be found in several tissues but PDE7 protein expression appears to be restricted [Han, P. et al. (1997); Perry, M.J. and G.A. Higgs (1998)]. Not much is known about the physiological function of PDE7.

The PDE8 family is closely related to the the PDE4 family. PDE8s have been shown to hydrolyze both cAMP and cGMP and are insensitive to inhibitors specific for PDEs 1-5. PDE8s are found in thyroid gland, testis, eye, liver, skeletal muscle, heart, kidney, ovary, and brain.

The PDE9 family preferentially hydrolyzes cAMP and is not sensitive to inhibition by rolipram, a PDE4-specific inhibitor, or isobutyl methyl xanthine (IBMX), a non-specific PDE inhibitor. PDE9 expression has been demonstrated in kidney, liver, lung, brain, spleen, and small intestine. Depending on nomenclature used, PDE9 is also referred to as PDE8, but is distinct from PDE8 mentioned above. To date, two genes have been identified in the PDE9 family.

PDE10 family members hydrolyze both cAMP and cGMP. PDE10s show expression in brain, thyroid and testis. [Soderling, S.H. et al. (1999); Fujishige, K. et al. (1999); Loughney, K. et al (1999)]

Members of the recently identified PDE11 family are also dualspecific. Interestingly, PDE11 splice variants exhibit different regulatory sequences in the N-terminal region. This suggests the possibility of differential regulation of PDE11s [Hetman JM, Robas N, Baxendale R, Fidock M, Phillips SC, Soderling SH, Beavo JA (2000)].

Increased PDE activity and decreased levels of cyclic nucleotides have been shown to be associated with many diseases. Furthermore, specific and non-specific inhibitors of several PDE protein families have been shown to be effective in treating such disorders. For example, the PDE4-specific inhibitor rolipram, mentioned above as an anti-depressant, inhibits lipopolysaccharide-induced expression of TNF-$\alpha$, and has been effective in treating multiple sclerosis in an animal model. Other PDE4-specific inhibitors are being investigated for use as anti-inflammatory therapeutics, and efficacy in attenuating the late asthmatic response to allergen challenge has been demonstrated [Harbinson, et al. (1997)]. Inhibitors specific for the PDE3 family have been approved for treatment of congestive heart failure. PDE5 inhibitors such as Sildenafil are in use for treatment of penile erectile dysfunction [Terrett, N. et al. (1996)]. PDE5-inhibitors are under investigation as agents for cardiovascular therapy [Perry, M.J. and G.A. Higgs (1998)].

PDEs cyclic nucleotide levels have been suggested to influence proliferation of different cell types [Conti et al. (1995)]. For example, growth of the prostatic carcinoma cell lines DU145 and LNCaP was inhibited by cAMP derivatives and PDE inhibitors [Bang, Y.J. et al. (1994)]. Furthermore, PDEs have been implemented to additional cancers.

Non-specific inhibitors, such as theophylline and pentoxifylline, are currently used in the treatment of respiratory and vascular disorders, respectively.

In summary, cAMP and cGMP play a central role in intracellular second messenger signaling. Furthermore, the value as pharmaceutical targets has been proven for several PDEs. Selective inhibitors have been developed as therapeutic agents for diseases such as cancer, heart failure, depression and sexual disfunction. Thus, the identification of further disease implications of PDE species and their splice variants may lead to the development of specific inhibitors or modulators, or suggest new utilities for known compounds affecting PDEs. That in turn will provide additional pharmacological approaches to treat diseases and conditions in which alterations in cyclic nucleotide pathways are involved. This diseases may include, but are not limited to, infections such as bacterial, fungal, protozoan, and viral infections, particularly those caused by HIV viruses, cancers, allergies including asthma, cardiovascular diseases including acute heart failure, hypotension, hypertension, angina pectoris, myocardial infarction, hematological diseases, genito-urinary diseases including urinary incontinence and benign prostate hyperplasia, osteoporosis, and peripheral and central nervous system disorders including pain, Alzheimer's disease and Parkinson's disease.

**TaqMan-Technology / expression profiling**

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up- or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g., drugs) may serve as indirect clues about disease-causing genes or drug targets. Moreover, the effects of drugs with established efficacy on global gene expression patterns may provide a guidepost, or a genetic signature, against which a new drug candidate can be compared.

## PDE4C

The cAMP-specific phosphodiesterase 4 (PDE4) is known to be mainly localized in inflammatory and immune cells as well as in smooth muscle cells of the bronchii. Isoenzyme-specific inhibitors (specific PDE4 inhibitors) have been developed [Huang Z et al., 2001], which show anti-inflammatory and bronchodilatatory activity in respective animal models. Some of these drugs are currently in clinical development for the indications asthma, chronic obstructive lung disease (COPD) and atopic dermatitis. A main problem which occurred during the clinical development of PDE4 inhibitors was nausea and emesis which is supposed to be evoked by PDE4 inhibition in brain tissue.

PDE4 itself can be subdivided in several subclasses, named PDE4A to PDE4D. One of the most famous PDE4 inhibitors is rolipram which often serves as tool in experimental studies. Rolipram specifically inhibits PDE4 (about 100fold selectivity against other PDEs, [Dym O et al., 2001]), which however does not discriminate between the different PDE4 subtypes. Recent efforts are directed to synthesize compounds which are specific to a discrete PDE4 subtype in order to specifically interfere with the physiologic or pathophysiologic role of this particular PDE4 subtype. Probably, it will be possible to prevent some of the adverse events in clinical development by synthesizing subtype-specific inhibitors.

The nucleotide sequence of PDE4C is accessible in public databases by the accession number NM_000923 and is given in SEQ ID NO: 1. The amino acid sequence of PDE4C is depicted in SEQ ID NO: 2.

PDE4C is described as a phosphodiesterase [Perry MJ et al., 1997]. The phosphodiesterase PDE4C is published in [Shelton et al., 1997], US 5686286, US 5672509 and WO9620281. The expression of PDE4C in testis and lung was previously described [Shelton et al., 1997]. PDE4C shows the highest homology to other phosphodiesterases as shown in example 1.

## Summary of the invention

The invention relates to novel disease associations of PDE4C polypeptides and polynucleotides. The invention also relates to novel methods of screening for therapeutic agents for the treatment of disorders of the peripheral and central nervous system, cardiovascular diseases (in particular angina and coronary heart disease), hematological diseases, inflammation, cancer, gastroenterological diseases and respiratory disease in a mammal. The invention also relates to pharmaceutical compositions for the treatment of disorders of the peripheral and central nervous system, cardiovascular diseases (in particular angina and coronary heart disease), hematological diseases, inflammation, cancer, gastroenterological diseases and respiratory disease in a mammal comprising a PDE4C polypeptide, a PDE4C polynucleotide, or regulators of PDE4C or modulators of PDE4C activity. The invention further comprises methods of diagnosing disorders of the peripheral and central nervous system, cardiovascular diseases (in particular angina and coronary heart disease), hematological diseases, inflammation, cancer, gastroenterological diseases and respiratory disease in a mammal.

## Brief Description of the Drawings

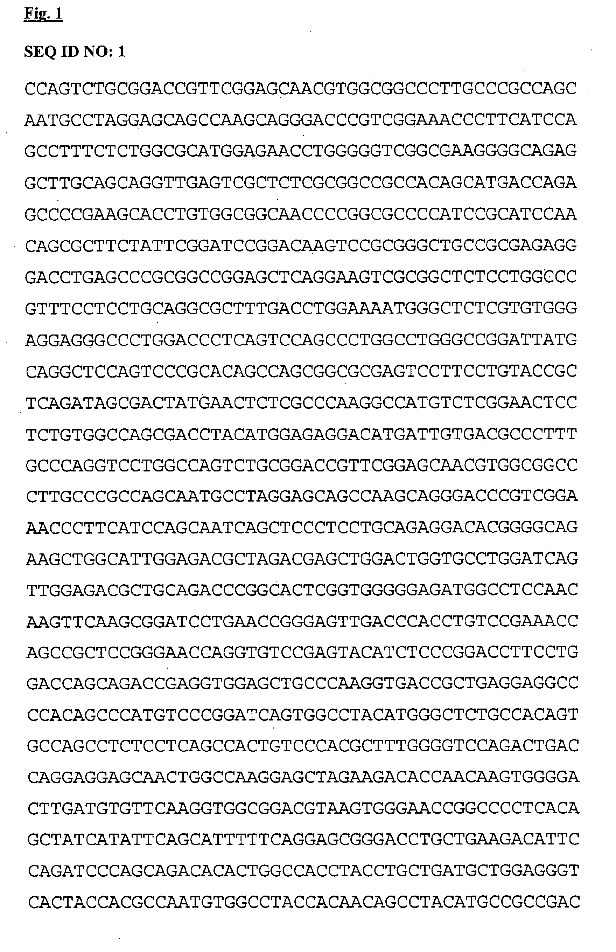

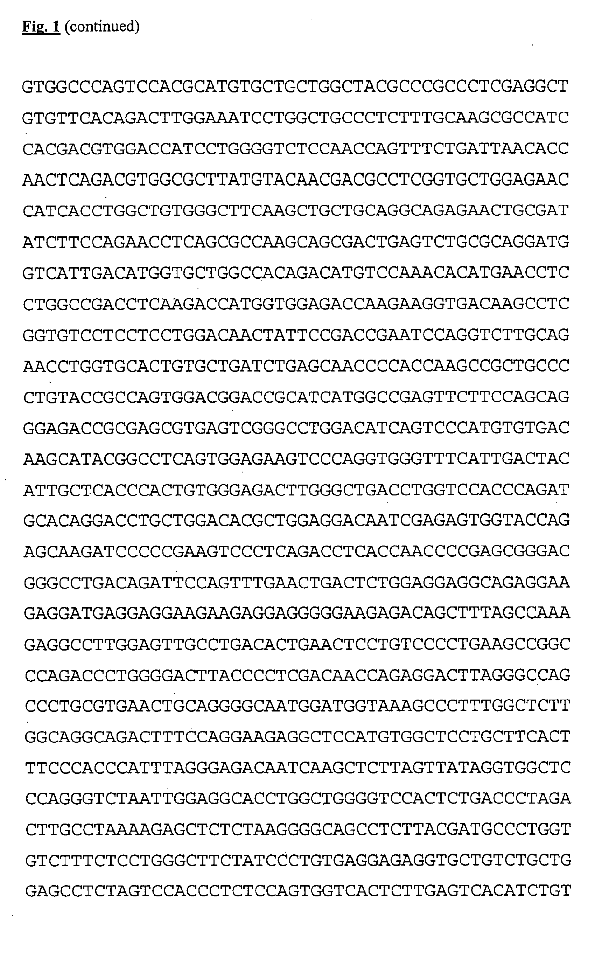

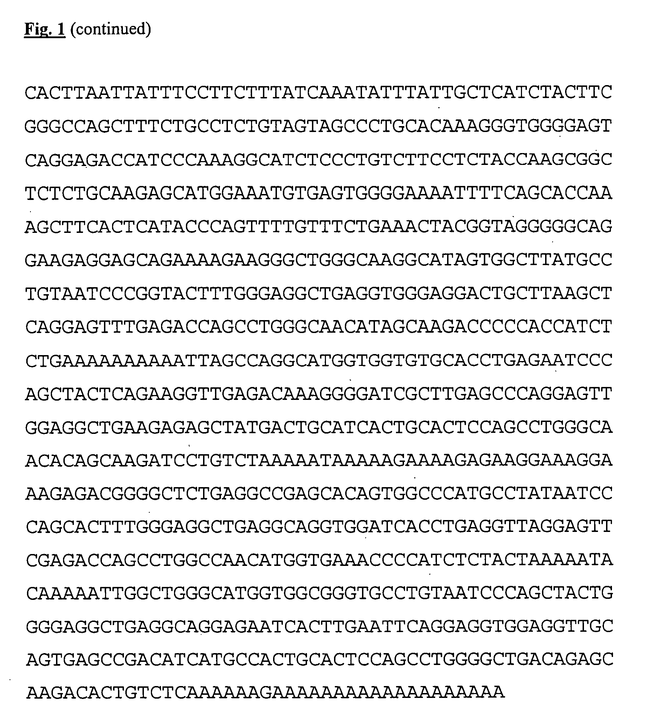

Fig. 1 shows the nucleotide sequence of a PDE4C polynucleotide (SEQ ID NO: 1).

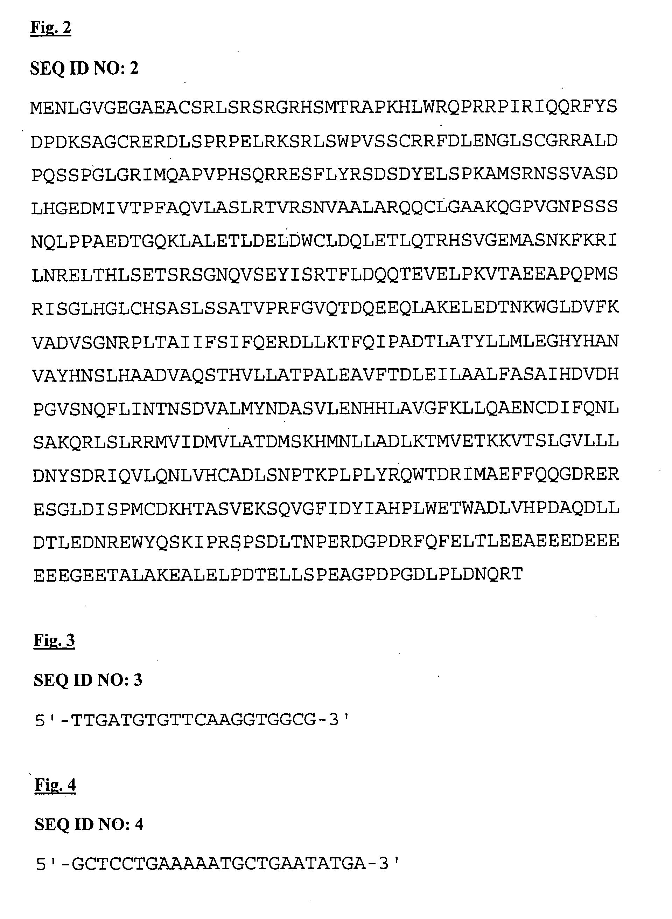

Fig. 2 shows the amino acid sequence of a PDE4C polypeptide (SEQ ID NO: 2).

Fig. 3 shows the nucleotide sequence of a primer useful for the invention (SEQ ID NO: 3).

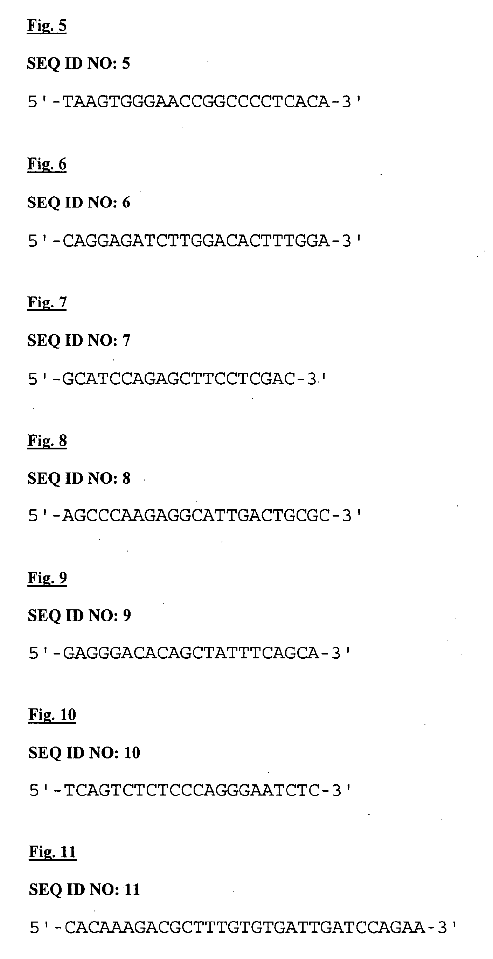

Fig. 4 shows the nucleotide sequence of a primer useful for the invention (SEQ ID NO: 4).

Fig. 5 shows a nucleotide sequence useful as a probe to detect proteins of the invention (SEQ ID NO: 5).

Fig. 6 shows the nucleotide sequence used as a forward primer for TaqMan experiments to detect PDE4A (SEQ ID NO: 6).

Fig. 7 shows the nucleotide sequence used as a reverse primer for TaqMan experiments to detect PDE4A (SEQ ID NO: 7).

Fig. 8 shows the nucleotide sequence used as a probe for TaqMan experiments to detect PDE4A (SEQ ID NO: 8).

Fig. 9 shows the nucleotide sequence used as a forward primer for TaqMan experiments to detect PDE4B (SEQ ID NO: 9).

Fig. 10 shows the nucleotide sequence used as a reverse primer for TaqMan experiments to detect PDE4B (SEQ ID NO: 10).

Fig. 11 shows the nucleotide sequence used as a probe for TaqMan experiments to detect PDE4B (SEQ ID NO: 11).

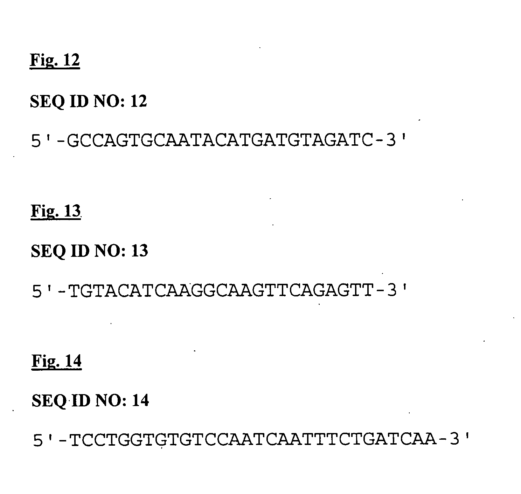

Fig. 12 shows the nucleotide sequence used as a forward primer for TaqMan experiments to detect PDE4D (SEQ ID NO: 12).

Fig. 13 shows the nucleotide sequence used as a reverse primer for TaqMan experiments to detect PDE4D (SEQ ID NO: 13).

Fig. 14 shows the nucleotide sequence used as a probe for TaqMan experiments to detect PDE4D (SEQ ID NO: 14).

## Detailed description of the invention

### Definition of terms

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A "fragment of a polynucleotide" is a nucleic acid that comprises all or any part of a given nucleotide molecule, the fragment having fewer nucleotides than about 6 kb, preferably fewer than about 1 kb.

"Reporter molecules" are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Chimeric" molecules may be constructed by introducing all or part of the nucleotide sequence of this invention into a vector containing additional nucleic acid sequence which might be expected to change any one or several of the following PDE4C characteristics: cellular location, distribution, ligand-binding affinities, interchain affinities, degradation/turnover rate, signaling, etc.

"Active", with respect to a PDE4C polypeptide, refers to those forms, fragments, or domains of a PDE4C polypeptide which retain the biological and/or antigenic activity of a PDE4C polypeptide.

"Naturally occurring PDE4C polypeptide" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Conservative amino acid substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal sequence" or "leader sequence" can be used, when desired, to direct the polypeptide through a membrane of a cell. Such a sequence may be naturally present on the polypeptides of the present invention or provided from heterologous sources by recombinant DNA techniques.

An "oligopeptide" is a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

"Inhibitor" is any substance which retards or prevents a chemical or physiological reaction or response. Common inhibitors include but are not limited to antisense molecules, antibodies, and antagonists.

"Standard expression" is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (e.g., cats, dogs, etc.), agricultural (e.g., cows, horses, sheep, etc.) or test species (e.g., mouse, rat, rabbit, etc.).

A "PDE4C polynucleotide", within the meaning of the invention, shall be understood as being a nucleic acid molecule selected from a group consisting of

(i) nucleic acid molecules encoding a polypeptide comprising the amino acid sequence of SEQ ID NO: 2,

(ii) nucleic acid molecules comprising the sequence of SEQ ID NO: 1,

(iii) nucleic acid molecules having the sequence of SEQ ID NO: 1,

(iv) nucleic acid molecules the complementary strand of which hybridizes under stringent conditions to a nucleic acid molecule of (i), (ii), or (iii); and

(v) nucleic acid molecules the sequence of which differs from the sequence of a nucleic acid molecule of (iii) due to the degeneracy of the genetic code;

wherein the polypeptide encoded by said nucleic acid molecule has PDE4C activity.

A "PDE4C polypeptide", within the meaning of the invention, shall be understood as being a polypeptide selected from a group consisting of

(i) polypeptides having the sequence of SEQ ID NO: 2,

(ii) polypeptides comprising the sequence of SEQ ID NO: 2,

(iii) polypeptides encoded by PDE4C polynucleotides; and

(iv) polypeptides which show at least 99%, 98%, 95%, 90%, or 80% homology with a polypeptide of (i), (ii), or (iii);

wherein said polypeptide has PDE4C activity.

The nucleotide sequences encoding a PDE4C (or their complement) have numerous applications in techniques known to those skilled in the art of molecular biology. These techniques include use as hybridization probes, use in the construction of oligomers for PCR, use for chromosome and gene mapping, use in the recombinant production of PDE4C, and use in generation of antisense DNA or RNA, their chemical analogs and the like. Uses of nucleotides encoding a PDE4C disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of PDE4C - encoding nucleotide sequences may be produced. Some of these will only bear minimal homology to the nucleotide sequence of the known and naturally occurring PDE4C. The invention has specifically contemplated each and every possible variation of nucleotide sequence that could be made by selecting combinations based on possible codon choices. These combinations are made in accordance with the standard triplet genetic code as applied to the nucleotide sequence of naturally occurring PDE4C, and all such variations are to be considered as being specifically disclosed.

Although the nucleotide sequences which encode a PDE4C, its derivatives or its variants are preferably capable of hybridizing to the nucleotide sequence of the naturally occurring PDE4C polynucleotide under stringent conditions, it may be advantageous to produce nucleotide sequences encoding PDE4C polypeptides or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding a PDE4C polypeptide and/or its derivatives without altering the encoded amino acid sequence include the production of RNA transcripts having more desirable properties, such as a greater half-life, than transcripts produced from the naturally occurring sequence.

Nucleotide sequences encoding a PDE4C polypeptide may be joined to a variety of other nucleotide sequences by means of well established recombinant DNA techniques. Useful nucleotide sequences for joining to PDE4C polynucleotides include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the subject invention is to provide for PDE4C-specific hybridization probes capable of hybridizing with naturally occurring nucleotide sequences encoding PDE4C. Such probes may also be used for the detection of similar PDE encoding sequences and should preferably show at least 40% nucleotide identity to PDE4C polynucleotides. The hybridization probes of the subject invention may be derived from the nucleotide sequence presented as SEQ ID NO: 1 or from genomic sequences including promoter, enhancers or introns of the native gene. Hybridization probes may be labelled by a variety of reporter molecules using techniques well known in the art.

It will be recognized that many deletional or mutational analogs of PDE4C polynucleotides will be effective hybridization probes for PDE4C polynucleotides. Accordingly, the invention relates to nucleic acid sequences that hybridize with such PDE4C encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes. Stringent conditions, within the meaning of the invention are 65°C in a buffer containing 1 mM EDTA, 0.5 M $NaHPO_4$ (pH 7.2), 7 % (w/v) SDS.

Nucleic acid molecules that will hybridize to PDE4C polynucleotides under stringent conditions can be identified functionally. Without limitation, examples of the uses for hybridization probes include: histochemical uses such as identifying tissues that express PDE4C; measuring mRNA levels, for instance to identify a sample's tissue type or to identify cells that express abnormal levels of PDE4C; and detecting polymorphisms of PDE4C.

PCR provides additional uses for oligonucleotides based upon the nucleotide sequence which encodes PDE4C. Such probes used in PCR may be of recombinant origin, chemically synthesized, or a mixture of both. Oligomers may comprise discrete nucleotide sequences employed under optimized conditions for identification of PDE4C in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction (PCR) primers are now established, as reviewed by PCR Protocols. Degenerate primers, i.e., preparations of primers that are heterogeneous at given sequence locations, can be designed to amplify nucleic acid sequences that are highly homologous to, but not identical with PDE4C. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplifications, particularly under preferred stringent hybridization conditions, are well known.

Other means of producing specific hybridization probes for PDE4C include the cloning of nucleic acid sequences encoding PDE4C or PDE4C derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

PDE4C polynucleotides may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

**Quantitative determinations of nucleic acids**

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson, (1989)], or hyper-variable minisatellite DNA [Barnes, 2000]. In recent years LOH has been performed primarily using PCR amplification of microsatellite markers and electrophoresis of the radio labelled [Jeffreys, (1985)] or fluorescently labelled PCR products [Weber, (1990)] and compared between paired normal and disease DNAs.

A number of other methods have also been developed to quantify nucleic acids [Gergen, (1992)]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas, (1980)].

A gene sequence contained in all samples at relatively constant quantity is typically utilized for sample amplification efficiency normalization. This approach, however, suffers from several drawbacks. The method requires that each sample has equal input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Piatak, (1993), BioTechniques]. The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecule.

*5' Fluorogenic Nuclease Assays*

Fluorogenic nuclease assays are a real time quantitation method that uses a probe to monitor formation of amplification product. The basis for this method of monitoring the formation of amplification product is to measure continuously PCR product accumulation using a dual-labelled fluorogenic oligonucleotide probe, an approach frequently referred to in the literature simply as the "TaqMan method" [Piatak, (1993), Science; Heid, (1996); Gibson, (1996); Holland. (1991)].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber optics connected with each well in a 96-or 384 well PCR tube arrangement. The instrument includes a laser for exciting the labels and is capable of measuring the fluorescence spectra intensity from each tube with continuous monitoring during PCR amplification. Each tube is re-examined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

*Non-Probe-Based Detection Methods*

A variety of options are available for measuring the amplification products as they are formed. One method utilizes labels, such as dyes, which only bind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium iodide, chromomycin, acridine orange, Hoechst 33258, Toto-1, Yoyo-1, DAPI (4',6-diamidino-2-phenylindole hydrochloride).

Another real time detection technique measures alteration in energy fluorescence energy transfer between fluorophors conjugated with PCR primers [Livak, (1995)].

*Probe-Based Detection Methods*

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from an oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

*Probes*

The labeled probe is selected so that its sequence is substantially complementary to a segment of the test locus or a reference locus. As indicated above, the nucleic acid site to which the probe binds should be located between the primer binding sites for the upstream and downstream amplification primers.

*Primers*

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe. Typically the primer includes 15-30 nucleotides. However, the length of the primer may be more or less depending on the complexity of the primer binding site and the factors listed above.

*Labels for Probes and Primers*

The labels used for labeling the probes or primers of the current invention and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or primer. These spacer arms are useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

**Patients Exhibiting Symptoms of Disease**

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

**PDE4C expression**

*PDE4C fusion proteins*

Fusion proteins are useful for generating antibodies against PDE4C polypeptides and for use in various assay systems. For example, fusion proteins can be used to identify proteins which interact with portions of PDE4C polypeptides. Protein affinity chromatography or library-based assays for protein-protein interactions, such as the yeast two-hybrid or phage display systems, can be used for this purpose. Such methods are well known in the art and also can be used as drug screens.

A PDE4C fusion protein comprises two polypeptide segments fused together by means of a peptide bond. The first polypeptide segment can comprise at least 54, 75, 100, 125, 139, 150, 175, 200, 225, 250, 275, 300, 325 or 350 contiguous amino acids of SEQ ID NO: 2 or of a biologically active variant, such as those described above. The first polypeptide segment also can comprise full-length PDE4C.

The second polypeptide segment can be a full-length protein or a protein fragment. Proteins commonly used in fusion protein construction include, but are not limited to β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located adjacent to the PDE4C.

*Preparation of Polynucleotides*

A naturally occurring PDE4C polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated PDE4C polynucleotides.

For example, restriction enzymes and probes can be used to isolate polynucleotide fragments which comprise PDE4C nucleotide sequences. Isolated polynucleotides are in preparations which are free or at least 70, 80, or 90% free of other molecules.

PDE4C cDNA molecules can be made with standard molecular biology techniques, using PDE4C mRNA as a template. PDE4C cDNA molecules can thereafter be replicated using molecular biology techniques known in the art. An amplification technique, such as PCR, can be used to obtain additional copies of polynucleotides of the invention, using either human genomic DNA or cDNA as a template.

Alternatively, synthetic chemistry techniques can be used to synthesizes PDE4C polynucleotides. The degeneracy of the genetic code allows alternate nucleotide sequences to be synthesized which will encode PDE4C having, for example, an amino acid sequence shown in SEQ ID NO: 2 or a biologically active variant thereof.

*Extending Polynucleotides*

Various PCR-based methods can be used to extend nucleic acid sequences encoding human PDE4C, for example to detect upstream sequences of PDE4C gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d (T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software (e.g., GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

PDE4C can be obtained, for example, by purification from human cells, by expression of PDE4C polynucleotides, or by direct chemical synthesis.

*Protein Purification*

PDE4C can be purified from any human cell which expresses the enzyme, including those which have been transfected with expression constructs which express PDE4C. A purified PDE4C is separated from other compounds which normally associate with PDE4C in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

*Expression of PDE4C Polynucleotides*

To express PDE4C, PDE4C polynucleotides can be inserted into an expression vector which contains the necessary elements for the transcription and translation of the inserted coding sequence. Methods which are well known to those skilled in the art can be used to construct expression vectors containing sequences encoding PDE4C and appropriate transcriptional and translational control elements. These methods include *in vitro* recombinant DNA techniques, synthetic techniques, *and in vivo* genetic recombination.

A variety of expression vector/host systems can be utilized to contain and express sequences encoding PDE4C. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.,* cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.*, heat shock, RUBISCO, and storage protein genes) or from plant viruses (*e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding PDE4C, vectors based on SV40 or EBV can be used with an appropriate selectable marker.

*Bacterial and Yeast Expression Systems*

In bacterial systems, a number of expression vectors can be selected. For example, when a large quantity of PDE4C is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding PDE4C can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

*Plant and Insect Expression Systems*

If plant expression vectors are used, the expression of sequences encoding PDE4C can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection.

An insect system also can be used to express PDE4C. For example, in one such system *Autographa californica* nuclear polyhedrosis virus (AcNPV) is used as a vector to express foreign genes in *Spodoptera frugiperda* cells or in *Trichoplusia* larvae. Sequences encoding PDE4C can be cloned into a non-essential region of the virus, such as the polyhedrin gene, and placed under control of the polyhedrin promoter. Successful insertion of PDE4C will render the polyhedrin gene inactive and produce recombinant virus lacking coat protein. The recombinant viruses can then be used to infect *S. frugiperda cells* or *Trichoplusia* larvae in which PDE4C can be expressed.

*Mammalian Expression Systems*

A number of viral-based expression systems can be used to express PDE4C in mammalian host cells. For example, if an adenovirus is used as an expression vector, sequences encoding PDE4C can be ligated into an adenovirus transcription/translation complex comprising the late promoter and tripartite leader sequence. Insertion in a non-essential E1 or E3 region of the viral genome can be used to obtain a viable virus which is capable of expressing PDE4C in infected host cells [Engelhard, 1994)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles). Specific initiation signals also can be used to achieve more efficient translation of sequences encoding PDE4C. Such signals include the ATG initiation codon and adjacent sequences. In cases where sequences encoding PDE4C, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

*Host Cells*

A host cell strain can be chosen for its ability to modulate the expression of the inserted sequences or to process the expressed PDE4C in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express PDE4C can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced PDE4C sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan, (1984)] and adenine phosphoribosyltransferase [Wigler, (1977)] genes which can be employed in *tk*$^-$ or *aprt*$^-$ cells, respectively. Also, antimetabolite, antibiotic, or herbicide resistance can be used as the basis for selection. For example, *dhfr* confers resistance to methotrexate [Lowy, (1980)], *npt* confers resistance to the aminoglycosides, neomycin and G-418 [Wigler, (1980)], and *als* and *pat* confer resistance to chlorsulfuron and phosphinotricin acetyltransferase, respectively [Colbere-Garapin, 1981]. Additional selectable genes have been described. For example, *trpB* allows cells to utilize indole in place of tryptophan, or *hisD*, which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system

*Detecting Polypeptide Expression*

Although the presence of marker gene expression suggests that a PDE4C polynucleotide is also present, its presence and expression may need to be confirmed. For example, if a sequence encoding PDE4C is inserted within a marker gene sequence, transformed cells containing sequences which encode PDE4C can be identified by the absence of marker gene function. Alternatively, a marker gene can be placed in tandem with a sequence encoding PDE4C under the control of a single promoter. Expression of the marker gene in response to induction or selection usually indicates expression of PDE4C polynucleotide.

Alternatively, host cells which contain a PDE4C polynucleotide and which express PDE4C can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding PDE4C can be detected by DNA-DNA or DNA-RNA hybridization or amplification using probes or fragments or fragments of polynucleotides encoding PDE4C. Nucleic acid amplification-based assays involve the use of oligonucleotides selected from sequences encoding PDE4C to detect transformants which contain a PDE4C polynucleotide.

A variety of protocols for detecting and measuring the expression of PDE4C, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on PDE4C can be used, or a competitive binding assay can be employed.

A wide variety of labels and conjugation techniques are known by those skilled in the art and can be used in various nucleic acid and amino acid assays. Means for producing labeled hybridization or PCR probes for detecting sequences related to polynucleotides encoding PDE4C include oligolabeling, nick translation, end-labeling, or PCR amplification using a labeled nucleotide. Alternatively, sequences encoding PDE4C can be cloned into a vector for the production of an mRNA probe. Such vectors are known in the art, are commercially available, and can be used to synthesize RNA *probes in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with PDE4C polynucleotides can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing PDE4C polynucleotides can be designed to contain signal sequences which direct secretion of soluble PDE4C through a prokaryotic or eukaryotic cell membrane or which direct the membrane insertion of membrane-bound PDE4C.

As discussed above, other constructions can be used to join a sequence encoding PDE4C to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and PDE4C also can be used to facilitate purification. One such expression vector provides for expression of a fusion protein containing PDE4C and 6 histidine residues preceding a thioredoxin or an enterokinase cleavage site. The histidine residues facilitate purification by IMAC (immobilized metal ion affinity chromatography) Maddox, (1983)], while the enterokinase cleavage site provides a means for purifying PDE4C from the fusion protein [Porath, (1992)].

*Chemical Synthesis*

Sequences encoding PDE4C can be synthesized, in whole or in part, using chemical methods well known in the art. Alternatively, PDE4C itself can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of PDE4C can be separately synthesized and combined using chemical methods to produce a full-length molecule.

The newly synthesized peptide can be substantially purified by preparative high performance liquid chromatography. The composition of a synthetic PDE4C can be confirmed by amino acid analysis or sequencing. Additionally, any portion of the amino acid sequence of PDE4C can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce PDE4C polynucleotides possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.
The nucleotide sequences referred to herein can be engineered using methods generally known in the art to alter PDE4C polynucleotides for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of PDE4C.
"Antibody" as used herein includes intact immunoglobulin molecules, as well as fragments thereof, such as Fab, $F(ab')_2$, and Fv, which are capable of binding an epitope of PDE4C. Typically, at least 6, 8, 10, or 12 contiguous amino acids are required to form an epitope. However, epitopes which involve non-contiguous amino acids may require more, e.g., at least 15, 25, or 50 amino acid. An antibody which specifically binds to an epitope of PDE4C can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the PDE4C immunogen.
Typically, an antibody which specifically binds to PDE4C provides a detection signal at least 5-, 10-, or 20-fold higher than a detection signal provided with other proteins when used in an immunochemical assay. Preferably, antibodies which specifically bind to PDE4C do not detect other proteins in immunochemical assays and can immunoprecipitate PDE4C from solution.
PDE4C can be used to immunize a mammal, such as a mouse, rat, rabbit, guinea pig, monkey, or human, to produce polyclonal antibodies. If desired, PDE4C can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e. g., aluminum hydroxide), and surface active substances (e.g., lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.
Monoclonal antibodies which specifically bind to PDE4C can be prepared using any technique which provides for the production of antibody molecules by continuous cell lines in culture. These techniques include, but are not limited to, the hybridoma technique, the human B-cell hybridoma technique, and the EBV-hybridoma technique [Roberge, (1995)].
In addition, techniques developed for the production of "chimeric antibodies", the splicing of mouse antibody genes to human antibody genes to obtain a molecule with appropriate antigen specificity and biological activity, can be used. Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Antibodies which specifically bind to PDE4C can contain antigen binding sites which are either partially or fully humanized, as disclosed in U.S. 5,565,332.
Alternatively, techniques described for the production of single chain antibodies can be adapted using methods known in the art to produce single chain antibodies which specifically bind to PDE4C. Antibodies with related specificity, but of distinct idiotypic composition, can be generated by chain shuffling from random combinatorial immunoglobin libraries. Single-chain antibodies also can be constructed using a DNA amplification method, such as PCR, using hybridoma cDNA as a template. Single-chain antibodies can be mono- or bispecific, and can be bivalent or tetravalent.

Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to PDE4C also can be produced by inducing *in vivo* production in the lymphocyte population or by screening immunoglobulin libraries or panels of highly specific binding reagents. Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which PDE4C is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of PDE4C gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of PDE4C gene expression can be obtained by designing antisense oligonucleotides which will form duplexes to the control, 5', or regulatory regions of the PDE4C gene. Oligonucleotides derived from the transcription initiation site, *e.g.*, between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls, (1993)]. An antisense oligonucleotide also can be designed to block translation of mRNA by preventing the transcript from binding to ribosomes.

Precise complementarity is not required for successful complex formation between an antisense oligonucleotide and the complementary sequence of a PDE4C polynucleotide. Antisense oligonucleotides which comprise, for example, 2, 3, 4, or 5 or more stretches of contiguous nucleotides which are precisely complementary to a PDE4C polynucleotide, each separated by a stretch of contiguous nucleotides which are not complementary to adjacent PDE4C nucleotides, can provide sufficient targeting specificity for PDE4C mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular PDE4C polynucleotide sequence. Antisense oligonucleotides can be modified without affecting their ability to hybridize to a PDE4C polynucleotide. These modifications can be internal or at one or both ends of the antisense molecule. For example, inter-nucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art.

*Ribozymes*

Ribozymes are RNA molecules with catalytic activity [Uhlmann, (1987)]. Ribozymes can be used to inhibit gene function by cleaving an RNA sequence, as is known in the art. The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a PDE4C polynucleotide can be used to generate ribozymes which will specifically bind to mRNA transcribed from a PDE4C polynucleotide.

Methods of designing and constructing ribozymes which can cleave other RNA molecules in trans in a highly sequence specific manner have been developed and described in the art. For example, the cleavage activity of ribozymes can be targeted to specific RNAs by engineering a discrete "hybridization" region into the ribozyme. The hybridization region contains a sequence complementary to the target RNA and thus specifically hybridizes with the target RNA.

Specific ribozyme cleavage sites within a PDE4C RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate PDE4C RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease PDE4C expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

## Screening / Screening Assays

*Regulators*

Regulators as used herein, refer to compounds that affect the activity of PDE4C in vivo and/or in vitro. Regulators can be agonists and antagonists of PDE4C polypeptide and can be compounds that exert their effect on the PDE4C activity via the enzymatic activity, expression, post-translational modifications or by other means. Agonists of PDE4C are molecules which, when bound to PDE4C, increase or prolong the activity of PDE4C. Agonists of PDE4C include proteins, nucleic acids, carbohydrates, small molecules, or any other molecule which activate PDE4C. Antagonists of PDE4C are molecules which, when bound to PDE4C, decrease the amount or the duration of the activity of PDE4C. Antagonists include proteins, nucleic acids, carbohydrates, antibodies, small molecules, or any other molecule which decrease the activity of PDE4C.

The term "modulate", as it appears herein, refers to a change in the activity of PDE4C polypeptide. For example, modulation may cause an increase or a decrease in enzymatic activity, binding characteristics, or any other biological, functional, or immunological properties of PDE4C.

As used herein, the terms "specific binding" or "specifically binding" refer to that interaction between a protein or peptide and an agonist, an antibody, or an antagonist. The interaction is dependent upon the presence of a particular structure of the protein recognized by the binding molecule (i.e., the antigenic determinant or epitope). For example, if an antibody is specific for epitope "A" the presence of a polypeptide containing the epitope A, or the presence of free unlabeled A, in a reaction containing free labeled A and the antibody will reduce the amount of labeled A that binds to the antibody.

The invention provides methods (also referred to herein as "screening assays") for identifying compounds which can be used for the treatment of hematological and cardiovascular diseases, disorders of the peripheral and central nervous system, COPD, asthma, genito-urological disorders and inflammation diseases. The methods entail the identification of candidate or test compounds or agents (e.g., peptides, peptidomimetics, small molecules or other molecules) which bind to PDE4C and/or have a stimulatory or inhibitory effect on the biological activity of PDE4C or its expression and then determining which of these compounds have an effect on symptoms or diseases regarding the hematological and cardiovascular diseases, disorders of the peripheral and central nervous system, COPD, asthma, genito-urological disorders and inflammation diseases in an *in vivo* assay.

Candidate or test compounds or agents which bind to PDE4C and/or have a stimulatory or inhibitory effect on the activity or the expression of PDE4C are identified either in assays that employ cells which express PDE4C (cell-based assays) or in assays with isolated PDE4C (cell-free assays). The various assays can employ a variety of variants of PDE4C (e.g., full-length PDE4C, a biologically active fragment of PDE4C, or a fusion protein which includes all or a portion of PDE4C). Moreover, PDE4C can be derived from any suitable mammalian species (e.g., human PDE4C, rat PDE4C or murine PDE4C). The assay can be a binding assay entailing direct or indirect measurement of the binding of a test compound or a known PDE4C ligand to PDE4C. The assay can also be an activity assay entailing direct or indirect measurement of the activity of PDE4C. The assay can also be an expression assay entailing direct or indirect measurement of the expression of PDE4C mRNA or PDE4C protein. The various screening assays are combined with an *in vivo* assay entailing measuring the effect of the test compound on the symptoms of hematological and cardiovascular diseases, disorders of the peripheral and central nervous system, COPD, asthma, genito-urological disorders and inflammation diseases.

The present invention includes biochemical, cell free assays that allow the identification of inhibitors and agonists of PDEs suitable as lead structures for pharmacological drug development. Such assays involve contacting a form of PDE4C (e.g., full-length PDE4C, a biologically active fragment of PDE4C, or a fusion protein comprising all or a portion of PDE4C) with a test compound and determining the ability of the test compound to act as an antagonist (preferably) or an agonist of the enzymatic activity of PDE4C. In one embodiment, the assay includes monitoring the PDE activity of PDE4C by measuring the conversion of either cAMP or cGMP to its nucleoside monophosphate after contacting PDE4C with a test compound.

For example, cAMP and cGMP levels can be measured by the use of the tritium containing compounds $^3$HcAMP and $^3$HcGMP as described in [Hansen, R.S., and Beavo, J.A., PNAS USA1982;79: 2788-92]. To screen a compound pool comprised of a large number of compounds, the microtiter plate-based scintillation proximity assay (SPA) as described in [Bardelle, C. et al. (1999) Anal. Biochem. 275: 148-155] can be applied.

Alternatively, the phosphodiesterase activity of the recombinant protein can be assayed using a commercially available SPA kit (Amersham Pharmacia). The PDE enzyme hydrolyzes cyclic nucleotides, e.g. cAMP and cGMP to their linear counterparts. The SPA assay utilizes the tritiated cyclic nucleotides [$^3$H]cAMP or [$^3$H]cGMP, and is based upon the selective interaction of the tritiated non cyclic product with the SPA beads whereas the cyclic substrates are not effectively binding. Radiolabelled product bound to the scintillation beads generates light that can be analyzed in a scintillation counter.

In one embodiment, the invention provides assays for screening candidate or test compounds which bind to or modulate the activity of PDE4C. Such assays can employ full-length PDE4C, a biologically active fragment of PDE4C, or a fusion protein which includes all or a portion of PDE4C. As described in greater detail below, the test compound can be obtained by any suitable means, e.g., from conventional compound libraries.

Determining the ability of the test compound to modulate the activity of PDE4C can be accomplished, for example, by determining the ability of PDE4C to bind to or interact with a target molecule. The target molecule can be a molecule with which PDE4C binds or interacts with in nature. The target molecule can be a component of a signal transduction pathway which facilitates transduction of an extracellular signal. The target PDE4C molecule can be, for example, a second intracellular protein which has catalytic activity or a protein which facilitates the association of downstream signaling molecules with PDE4C.

Determining the ability of PDE4C to bind to or interact with a target molecule can be accomplished by one of the methods described above for determining direct binding. In one embodiment, determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular $Ca^{2+}$, diacylglycerol, $IP_3$, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g., luciferase), or detecting a cellular response.

In various embodiments of the above assay methods of the present invention, it may be desirable to immobilize PDE4C (or a PDE4C target molecule) to facilitate separation of complexed from uncomplexed forms of one or both of the proteins, as well as to accommodate automation of the assay. Binding of a test compound to PDE4C, or interaction of PDE4C with a target molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed target protein or PDE4C, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of PDE4C can be determined using standard techniques.

Other techniques for immobilizing proteins on matrices can also be used in the screening assays of the invention. For example, either PDE4C or its target molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated plates (Pierce Chemical). Alternatively, antibodies reactive with PDE4C or target molecules but which do not interfere with binding of the polypeptide of the invention to its target molecule can be derivatized to the wells of the plate, and unbound target or polypeptide of the invention trapped in the wells by antibody conjugation. Methods for detecting such complexes, in addition to those described above for the GST-immobilized complexes, include immunodetection of complexes using antibodies reactive with PDE4C or target molecule, as well as enzyme-linked assays which rely on detecting an enzymatic activity associated with PDE4C or target molecule.

Another technique for drug screening which may be used provides for high throughput screening of compounds having suitable binding affinity to the protein of interest as described in published PCT application WO84/03564. In this method, large numbers of different small test compounds are synthesized on a solid substrate, such as plastic pins or some other surface. The test compounds are reacted with PDE4C, or fragments thereof, and washed. Bound PDE4C is then detected by methods well known in the art. Purified PDE4C can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding PDE4C specifically compete with a testcompound for binding PDE4C. In this manner, antibodies can be used to detect the presence of any peptide which shares one or more antigenic determinants with PDE4C.

The screening assay can also involve monitoring the expression of PDE4C. For example, regulators of expression of PDE4C can be identified in a method in which a cell is contacted with a candidate compound and the expression of PDE4C protein or mRNA in the cell is determined. The level of expression of PDE4C protein or mRNA the presence of the candidate compound is compared to the level of expression of PDE4C protein or mRNA in the absence of the candidate compound. The candidate compound can then be identified as a regulator of expression of PDE4C based on this comparison. For example, when expression of PDE4C protein or mRNA protein is greater (statistically significantly greater) in the presence of the candidate compound than in its absence, the candidate compound is identified as a stimulator of PDE4C protein or mRNA expression. Alternatively, when expression of PDE4C protein or mRNA is less (statistically significantly less) in the presence of the candidate compound than in its absence, the candidate compound is identified as an inhibitor of PDE4C protein or mRNA expression. The level of PDE4C protein or mRNA expression in the cells can be determined by methods described below.

*Binding Assays*

For binding assays, the test compound is preferably a small molecule which binds to and occupies the active site of PDE4C polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known PDE4C PDEs and analogues or derivatives thereof.

In binding assays, either the test compound or the PDE4C polypeptide can comprise a detectable label, such as a fluorescent, radioisotopic, chemiluminescent, or enzymatic label, such as horseradish peroxidase, alkaline phosphatase, or luciferase. Detection of a test compound which is bound to PDE4C polypeptide can then be accomplished, for example, by direct counting of radioemmission, by scintillation counting, or by determining conversion of an appropriate substrate to a detectable product. Alternatively, binding of a test compound to a PDE4C polypeptide can be determined without labeling either of the interactants. For example, a microphysiometer can be used to detect binding of a test compound with a PDE4C polypeptide. A microphysiometer (*e.g.*, Cytosensor™) is an analytical instrument that measures the rate at which a cell acidifies its environment using a light-addressable potentiometric sensor (LAPS). Changes in this acidification rate can be used as an indicator of the interaction between a test compound and PDE4C [Haseloff, (1988)].

Determining the ability of a test compound to bind to PDE4C also can be accomplished using a technology such as real-time Bimolecular Interaction Analysis (BIA) [McConnell, (1992); Sjolander, (1991)]. BIA is a technology for studying bio-specific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, a PDE4C-like polypeptide can be used as a "bait protein" in a two-hybrid assay or three-hybrid assay [Szabo, (1995); U.S. 5,283,317), to identify other proteins which bind to or interact with PDE4C and modulate its activity.

The two-hybrid system is based on the modular nature of most transcription factors, which consist of separable DNA-binding and activation domains. Briefly, the assay utilizes two different DNA constructs. For example, in one construct, polynucleotide encoding PDE4C can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with PDE4C.

It may be desirable to immobilize either the PDE4C (or polynucleotide) or the test compound to facilitate separation of the bound form from unbound forms of one or both of the interactants, as well as to accommodate automation of the assay. Thus, either the PDE4C-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach PDE4C-like polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to PDE4C (or a polynucleotide encoding for PDE4C) can be accomplished in any vessel suitable for containing the reactants. Examples of such vessels include microtiter plates, test tubes, and microcentrifuge tubes.

In one embodiment, PDE4C is a fusion protein comprising a domain that allows binding of PDE4C to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed PDE4C; the mixture is then incubated under conditions conducive to complex formation (*e.g.*, at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either PDE4C (or a polynucleotide encoding PDE4C) or a test compound can be immobilized utilizing conjugation of biotin and streptavidin. Biotinylated PDE4C (or a polynucleotide encoding biotinylated PDE4C) or test compounds can be prepared from biotin-NHS (N-hydroxysuccinimide) using techniques well known in the art (*e.g.*, biotinylation kit, Pierce Chemicals, Rockford, Ill.) and immobilized in the wells of streptavidin-coated plates (Pierce Chemical). Alternatively, antibodies which specifically bind to PDE4C, polynucleotide, or a test compound, but which do not interfere with a desired binding site, such as the active site of PDE4C, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to PDE4C polypeptide or test compound, enzyme-linked assays which rely on detecting an activity of PDE4C polypeptide, and SDS gel electrophoresis under non-reducing conditions.

Screening for test compounds which bind to a PDE4C polypeptide or polynucleotide also can be carried out in an intact cell. Any cell which comprises a PDE4C polypeptide or polynucleotide can be used in a cell-based assay system. A PDE4C polynucleotide can be naturally occurring in the cell or can be introduced using techniques such as those described above. Binding of the test compound to PDE4C or a polynucleotide encoding PDE4C is determined as described above.

*Functional Assays*

Test compounds can be tested for the ability to increase or decrease PDE4C activity of a PDE4C polypeptide. The PDE4C activity can be measured, for example, using methods described in the specific examples, below. PDE4C activity can be measured after contacting either a purified PDE4C or an intact cell with a test compound. A test compound which decreases PDE4C activity by at least about 10, preferably about 50, more preferably about 75, 90, or 100% is identified as a potential agent for decreasing PDE4C activity. A test compound which increases PDE4C activity by at least about 10, preferably about 50, more preferably about 75, 90, or 100% is identified as a potential agent for increasing PDE4C activity.

*Gene Expression*

In another embodiment, test compounds which increase or decrease PDE4C gene expression are identified.

As used herein, the term "correlates with expression of a polynucleotide" indicates that the detection of the presence of nucleic acids, the same or related to a nucleic acid sequence encoding PDE4C, by northern analysis or realtime PCR is indicative of the presence of nucleic acids encoding PDE4C in a sample, and thereby correlates with expression of the transcript from the polynucleotide encoding PDE4C. The term "microarray", as used herein, refers to an array of distinct polynucleotides or oligonucleotides arrayed on a substrate, such as paper, nylon or any other type of membrane, filter, chip, glass slide, or any other suitable solid support. A PDE4C polynucleotide is contacted with a test compound, and the expression of an RNA or polypeptide product of PDE4C polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of PDE4C mRNA or polypeptide expression in the cells can be determined by methods well known in the art for detecting mRNA or polypeptide. Either qualitative or quantitative methods can be used. The presence of polypeptide products of PDE4C polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labelled amino acids into PDE4C.

Such screening can be carried out either in a cell-free assay system or in an intact cell. Any cell which expresses PDE4C polynucleotide can be used in a cell-based assay system. The PDE4C polynucleotide can be naturally occurring in the cell or can be introduced using techniques such as those described above. Either a primary culture or an established cell line can be used.

*Test Compounds*

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

*Modeling of Regulators*

Computer modeling and searching technologies permit identification of compounds, or the improvement of already identified compounds, that can modulate PDE4C expression or activity. Having identified such a compound or composition, the active sites or regions are identified. Such sites might typically be the enzymatic active site, regulator binding sites, or ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential PDE4C modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

**Therapeutic Indications and Methods**

It was found by the present applicant that PDE4C is expressed in various human tissues.

*Neurology*

CNS disorders include disorders of the central nervous system as well as disorders of the peripheral nervous system.

CNS disorders include, but are not limited to brain injuries, cerebrovascular diseases and their consequences, Parkinson's disease, corticobasal degeneration, motor neuron disease, dementia, including ALS, multiple sclerosis, traumatic brain injury, stroke, post-stroke, post-traumatic brain injury, and small-vessel cerebrovascular disease. Dementias, such as Alzheimer's disease, vascular dementia, dementia with Lewy bodies, frontotemporal dementia and Parkinsonism linked to chromosome 17, frontotemporal dementias, including Pick's disease, progressive nuclear palsy, corticobasal degeneration, Huntington's disease, thalamic degeneration, Creutzfeld-Jakob dementia, HIV dementia, schizophrenia with dementia, and Korsakoff's psychosis, within the meaning of the definition are also considered to be CNS disorders.

Similarly, cognitive-related disorders, such as mild cognitive impairment, age-associated memory impairment, age-related cognitive decline, vascular cognitive impairment, attention deficit disorders, attention deficit hyperactivity disorders, and memory disturbances in children with learning disabilities are also considered to be CNS disorders.

Pain, within the meaning of this definition, is also considered to be a CNS disorder. Pain can be associated with CNS disorders, such as multiple sclerosis, spinal cord injury, sciatica, failed back surgery syndrome, traumatic brain injury, epilepsy, Parkinson's disease, post-stroke, and vascular lesions in the brain and spinal cord (e.g., infarct, hemorrhage, vascular malformation). Non-central neuropathic pain includes that associated with post mastectomy pain, phantom feeling, reflex sympathetic dystrophy (RSD), trigeminal neuralgiaradioculopathy, post-surgical pain, HIV/AIDS related pain, cancer pain, metabolic neuropathies (e.g., diabetic neuropathy, vasculitic neuropathy secondary to connective tissue disease), paraneoplastic polyneuropathy associated, for example, with carcinoma of lung, or leukemia, or lymphoma, or carcinoma of prostate, colon or stomach, trigeminal neuralgia, cranial neuralgias, and post-herpetic neuralgia. Pain associated with peripheral nerve damage, central pain (i.e. due to cerebral ischemia) and various chronic pain i.e., lumbago, back pain (low back pain), inflammatory and/or rheumatic pain. Headache pain (for example, migraine with aura, migraine without aura, and other migraine disorders), episodic and chronic tension-type headache, tension-type like headache, cluster headache, and chronic paroxysmal hemicrania are also CNS disorders.

Visceral pain such as pancreatits, intestinal cystitis, dysmenorrhea, irritable Bowel syndrome, Crohn's disease, biliary colic, ureteral colic, myocardial infarction and pain syndromes of the pelvic cavity, e.g., vulvodynia, orchialgia, urethral syndrome and protatodynia are also CNS disorders.

Also considered to be a disorder of the nervous system are acute pain, for example postoperative pain, and pain after trauma.

The human PDE4C is highly expressed in the following brain tissues: cerebral cortex, Alzheimer cerebral cortex, occipital lobe, parietal lobe, temporal lobe, precentral gyrus, substantia nigra, hippocampus, thalamus, dorsal root ganglia. The expression in brain tissues and in particular the differential expression between diseased tissue Alzheimer cerebral cortex and healthy tissue cerebral cortex demonstrates that the human PDE4C or mRNA can be utilized to diagnose nervous system diseases. Additionally the activity of the human PDE4C can be modulated to treat nervous system diseases.

*Cardiovascular Disorders*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias, atrial tachycardia, atrial flutter, atrial fibrillation, atrio-ventricular reentrant tachycardia, preexitation syndrome, ventricular tachycardia, ventricular flutter, ventricular fibrillation, as well as bradycardic forms of arrhythmias.

Hypertensive vascular diseases include primary as well as all kinds of secondary arterial hypertension, renal, endocrine, neurogenic, others. The genes may be used as drug targets for the treatment of hypertension as well as for the prevention of all complications arising from cardiovascular diseases.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis is a cardiovascular disease in which the vessel wall is remodeled, compromising the lumen of the vessel. The atherosclerotic remodeling process involves accumulation of cells, both smooth muscle cells and monocyte/macrophage inflammatory cells, in the intima of the vessel wall. These cells take up lipid, likely from the circulation, to form a mature atherosclerotic lesion. Although the formation of these lesions is a chronic process, occurring over decades of an adult human life, the majority of the morbidity associated with atherosclerosis occurs when a lesion ruptures, releasing thrombogenic debris that rapidly occludes the artery. When such an acute event occurs in the coronary artery, myocardial infarction can ensue, and in the worst case, can result in death.

The formation of the atherosclerotic lesion can be considered to occur in five overlapping stages such as migration, lipid accumulation, recruitment of inflammatory cells, proliferation of vascular smooth muscle cells, and extracellular matrix deposition. Each of these processes can be shown to occur in man and in animal models of atherosclerosis, but the relative contribution of each to the pathology and clinical significance of the lesion is unclear.

Thus, a need exists for therapeutic methods and agents to treat cardiovascular pathologies, such as atherosclerosis and other conditions related to coronary artery disease.

Cardiovascular diseases include but are not limited to disorders of the heart and the vascular system like congestive heart failure, myocardial infarction, ischemic diseases of the heart, all kinds of atrial and ventricular arrhythmias, hypertensive vascular diseases, peripheral vascular diseases, and atherosclerosis.

Too high or to low levels of fats in the bloodstream, especially cholesterol, can cause long-term problems. The risk to develop atherosclerosis and coronary artery or carotid artery disease (and thus the risk of having a heart attack or stroke) increases with the total cholesterol level increasing. Nevertheless, extremely low cholesterol levels may not be healthy. Examples of disorders of lipid metabolism are hyperlipidemia (abnormally high levels of fats (cholesterol, triglycerides, or both) in the blood, may be caused by family history of hyperlipidemia), obesity, a high-fat diet, lack of exercise, moderate to high alcohol consumption, cigarette smoking, poorly controlled diabetes, and an underactive thyroid gland), hereditary hyper-lipidemias (type I hyperlipoproteinemia (familial hyperchylomicronemia), type II hyperlipoproteinemia (familial hypercholesterolemia), type III hyperlipoproteinemia, type IV hyperlipoproteinemia, or type V hyperlipoproteinemia), hypolipoproteinemia, lipidoses (caused by abnormalities in the enzymes that metabolize fats), Gaucher's disease, Niemann-Pick disease, Fabry's disease, Wolman's disease, cerebrotendinous xanthomatosis, sitosterolemia, Refsum's disease, or Tay-Sachs disease.

Kidney disorders may lead to hypertension or hypotension. Examples for kidney problems possibly leading to hypertension are renal artery stenosis, pyelonephritis, glomerulonephritis, kidney tumors, polycistic kidney disease, injury to the kidney, or radiation therapy affecting the kidney. Excessive urination may lead to hypotension.

The human PDE4C is highly expressed in the following cardiovascular related tissues: fetal heart, heart, pericardium, heart atrium (right), heart atrium (left), heart ventricle (left), interventricular septum, aorta sclerotic, artery, coronary artery, coronary artery sclerotic, coronary artery smooth muscle primary cells, HUVEC cells. Expression in the above mentioned tissues and in particular the differential expression between diseased tissue aorta sclerotic and healthy tissue aorta and coronary artery sclerotic and healthy tissue coronary artery demonstrates that the human PDE4C or mRNA can be utilized to diagnose of cardiovascular diseases.

Additionally the activity of the human PDE4C can be modulated to treat cardiovascular diseases.

It was shown in particular by the inventors, that inhibition of PDE4C activity is useful for the treatment of diseases involving ischemia, like angina and coronary heart disease, as disclosed in the following.

The role of PDE4 in the cardiovascular system is not investigated in detail. It is known, that in myocardial tissue all PDE4 subtypes are expressed [Ückert S et al., 2001]. The inventors were able to confirm this finding using human cardiac tissue. However, in contrast to published data we investigated myocardial tissue and the coronary arteries separately and we surprisingly found a different expression of PDE4-subtypes in human myocardium and coronary arteries (see example 2, table 2).

As can be seen from table 2, PDE4C is not the most abundant isoform in human cardiac tissue but in contrast to all other isoforms, PDE4C is the only subtype which is preferentially expressed in coronary arteries. This can also be seen in example 2, table 3 where the individual values of PDE4C expression of 5 different patients are shown. In 3 of 5 patients, expression of PDE4C is 10times higher in coronary arteries compared to myocardium.

In the current literature no relevant effects of PDE4 inhibition on the cardiovascular system are described. Simpson et al. investigated the effects of rolipram in anaesthetized dogs in an ischemia/reperfusion model [Simpson PJ et al., 1992]. Rolipram, using the high dose of 1 mg/kg iv + 1mg/kg/h infusion did not affect blood pressure and contractility.

We administered the same inhibitor (rolipram) using the very low doses (10-100 µg/kg/h) to anaesthetized dogs and investigated the cardiovascular effects (example 3). In contrast to the published data administration of rolipram at a dose of 30 µg/kg/h resulted in a slight increase in blood pressure, heart rate, contractility and coronary blood flow. These effects can be explained by an intracellular increase in cAMP in myocardial tissue; since the effects occur at very low doses it can be assumed that the effects are specific and most probably caused by PDE4 inhibition.

This finding is of particular relevance since the endogenous protective mechanisms (e.g. adenosine release under ischemic conditions) also act via an increase of cAMP. A low dose of a PDE4 inhibitor could act as a "drug on demand": Under normal conditions no cardiovascular effects will be seen. However, during ischemia (e.g. angina) the endogenous mechanisms which promote an increase of cAMP could be enhanced by inhibiting cAMP breakdown through PDE4 inhibition.

"Reactive hyperemia" can serve as a model for this pathophysiologic condition: in anaesthetized dogs (experimental setup: as in example 3) coronary blood flow is interrupted by short term ligation (4 min) of the coronary artery. Restoration of blood flow results in a reactive hyperemia: under control conditions blood flow increases by 106%, after 2.5 min the effect has subsided to 50%. During a low dose of rolipram which itself does not evoke cardiovascular effects (in this case 10µg/kg/h) the observed reactive hyperemia is more pronounced (increase in flow +174%) and continues for a longer time ($T_{1/2}$ = 5 min).

A PDE4 inhibitor, which does not pass the blood-brain-barrier and/or has no emetic potential (achieved by its subtype selectivity), is a new principle in coronary therapy. Since PDE4C is preferentially expressed in coronary arteries, a specific PDE4C inhibitor is very well suited to increase coronary blood flow by simultaneously minimizing the effects on heart rate.

These findings show that modulation of the PDE4C activity can be used to treat cardiovascular disorders. In particular, these findings demonstrate that inhibition of PDE4C activity is useful for the treatment of diseases involoving ischemia, like angina or coronary heart disease.

*Hematological Disorders*

Hematological disorders comprise diseases of the blood and all its constituents as well as diseases of organs and tissues involved in the generation or degradation of all the constituents of the blood. They include but are not limited to 1) Anemias, 2) Myeloproliferative Disorders, 3) Hemorrhagic Disorders, 4) Leukopenia, 5) Eosinophilic Disorders, 6) Leukemias, 7) Lymphomas, 8) Plasma Cell Dyscrasias, 9) Disorders of the Spleen in the course of hematological disorders. Disorders according to 1) include, but are not limited to anemias due to defective or deficient hem synthesis, deficient erythropoiesis. Disorders according to 2) include, but are not limited to polycythemia vera, tumor-associated erythrocytosis, myelofibrosis, thrombocythemia. Disorders according to 3) include, but are not limited to vasculitis, thrombocytopenia, heparin-induced thrombocytopenia, thrombotic thrombocytopenic purpura, hemolytic-uremic syndrome, hereditary and acquired disorders of platelet function, hereditary coagulation disorders. Disorders according to 4) include, but are not limited to neutropenia, lymphocytopenia. Disorders according to 5) include, but are not limited to hypereosinophilia, idiopathic hypereosinophilic syndrome. Disorders according to 6) include, but are not limited to acute myeloic leukemia, acute lymphoblastic leukemia, chronic myelocytic leukemia, chronic lymphocytic leukemia, myelodysplastic syndrome. Disorders according to 7) include, but are not limited to Hodgkin's disease, non-Hodgkin's lymphoma, Burkitt's lymphoma, mycosis fungoides cutaneous T-cell lymphoma. Disorders according to 8) include, but are not limited to multiple myeloma, macroglobulinemia, heavy chain diseases. In extension of the preceding idiopathic thrombocytopenic purpura, iron deficiency anemia, megaloblastic anemia (vitamin B12 deficiency), aplastic anemia, thalassemia, malignant lymphoma bone marrow invasion, malignant lymphoma skin invasion, hemolytic uremic syndrome, giant platelet disease are considered to be hematological diseases too.

The human PDE4C is highly expressed in the following tissues of the hematological system: erythrocytes, lymphnode, spleen, spleen liver cirrhosis. The expression in the above mentioned tissues and in particular the differential expression between diseased tissue spleen liver cirrhosis and healthy tissue spleen demonstrates that the human PDE4C or mRNA can be utilized to diagnose of hematological diseases. Additionally the activity of the human PDE4C can be modulated to treat hematological disorders.

*Gastrointestinal and Liver Diseases*

Gastrointestinal diseases comprise primary or secondary, acute or chronic diseases of the organs of the gastrointestinal tract which may be acquired or inherited, benign or malignant or metaplastic, and which may affect the organs of the gastrointestinal tract or the body as a whole. They comprise but are not limited to 1) disorders of the esophagus like achalasia, vigoruos achalasia, dysphagia, cricopharyngeal incoordination, pre-esophageal dysphagia, diffuse esophageal spasm, globus sensation, Barrett's metaplasia, gastroesophageal reflux, 2) disorders of the stomach and duodenum like functional dyspepsia, inflammation of the gastric mucosa, gastritis, stress gastritis, chronic erosive gastritis, atrophy of gastric glands, metaplasia of gastric tissues, gastric ulcers, duodenal ulcers, neoplasms of the stomach, 3) disorders of the pancreas like acute or chronic pancreatitis, insufficiency of the exocrinic or endocrinic tissues of the pancreas like steatorrhea, diabetes, neoplasms of the exocrine or endocrine pancreas like 3.1) multiple endocrine neoplasia syndrome, ductal adenocarcinoma, cystadenocarcinoma, islet cell tumors, insulinoma, gastrinoma, carcinoid tumors, glucagonoma, Zollinger-Ellison syndrome, Vipoma syndrome, malabsorption syndrome, 4) disorders of the bowel like chronic inflammatory diseases of the bowel, Crohn's disease, ileus, diarrhea and constipation, colonic inertia, megacolon, malabsorption syndrome, ulcerative colitis, 4.1) functional bowel disorders like irritable bowel syndrome, 4.2) neoplasms of the bowel like familial polyposis, adenocarcinoma, primary malignant lymphoma, carcinoid tumors, Kaposi's sarcoma, polyps, cancer of the colon and rectum.

Liver diseases comprise primary or secondary, acute or chronic diseases or injury of the liver which may be acquired or inherited, benign or malignant, and which may affect the liver or the body as a whole. They comprise but are not limited to disorders of the bilirubin metabolism, jaundice, syndroms of Gilbert's, Crigler-Najjar, Dubin-Johnson and Rotor; intrahepatic cholestasis, hepatomegaly, portal hypertension, ascites, Budd-Chiari syndrome, portal-systemic encephalopathy, fatty liver, steatosis, Reye's syndrome, liver diseases due to alcohol, alcoholic hepatitis or cirrhosis, fibrosis and cirrhosis, fibrosis and cirrhosis of the liver due to inborn errors of metabolism or exogenous substances, storage diseases, syndromes of Gaucher's, Zellweger's, Wilson's - disease, acute or chronic hepatitis, viral hepatitis and its variants, inflammatory conditions of the liver due to viruses, bacteria, fungi, protozoa, helminths; drug induced disorders of the liver, chronic liver diseases like primary sclerosing cholangitis, $alpha_1$-antitrypsin-deficiency, primary biliary cirrhosis, postoperative liver disorders like postoperative intrahepatic cholestasis, hepatic granulomas, vascular liver disorders associated with systemic disease, benign or malignant neoplasms of the liver, disturbance of liver metabolism in the new-born or prematurely born.

The human PDE4C is highly expressed in the following tissues of the gastroenterological system: stomach, stomach tumor, colon, colon tumor, ileum, ileum chronic inflammation, rectum, liver, liver liver cirrhosis, liver tumor. The expression in the above mentioned tissues and in particular the differential expression between diseased tissue stomach tumor and healthy tissue stomach demonstrates that the human PDE4C or mRNA can be utilized to diagnose of gastroenterological disorders. Additionally the activity of the human PDE4C can be modulated to treat gastroenterological disorders.

*Cancer Disorders*

Cancer disorders within the scope of this definition comprise any disease of an organ or tissue in mammals characterized by poorly controlled or uncontrolled multiplication of normal or abnormal cells in that tissue and its effect on the body as a whole. Cancer diseases within the scope of the definition comprise benign neoplasms, dysplasias, hyperplasias as well as neoplasms showing metastatic growth or any other transformations like e.g. leukoplakias which often precede a breakout of cancer. Cells and tissues are cancerous when they grow more rapidly than normal cells, displacing or spreading into the surrounding healthy tissue or any other tissues of the body described as metastatic growth, assume abnormal shapes and sizes, show changes in.their nucleocytoplasmatic ratio, nuclear polychromasia, and finally may cease. Cancerous cells and tissues may affect the body as a whole when causing paraneoplastic syndromes or if cancer occurs within a vital organ or tissue, normal function will be impaired or halted, with possible fatal results. The ultimate involvement of a vital organ by cancer, either primary or metastatic, may lead to the death of the mammal affected. Cancer tends to spread, and the extent of its spread is usually related to an individual's chances of surviving the disease. Cancers are generally said to be in one of three stages of growth: early, or localized, when a tumor is still confined to the tissue of origin, or primary site; direct extension, where cancer cells from the tumour have invaded adjacent tissue or have spread only to regional lymph nodes; or metastasis, in which cancer cells have migrated to distant parts of the body from the primary site, via the blood or lymph systems, and have established secondary sites of infection. Cancer is said to be malignant because of its tendency to cause death if not treated. Benign tumors usually do not cause death, although they may if they interfere with a normal body function by virtue of their location, size, or paraneoplastic side effects. Hence benign tumors fall under the definition of cancer within the scope of this definition as well. In general, cancer cells divide at a higher rate than do normal cells, but the distinction between the growth of cancerous and normal tissues is not so much the rapidity of cell division in the former as it is the partial or complete loss of growth restraint in cancer cells and their failure to differentiate into a useful, limited tissue of the type that characterizes the functional equilibrium of growth of normal tissue. Cancer tissues may express certain molecular receptors and probably are influenced by the host's susceptibility and immunity and it is known that certain cancers of the breast and prostate, for example, are considered dependent on specific hormones for their existence. The term "cancer" under the scope of the definition is not limited to simple benign neoplasia but comprises any other benign and malign neoplasia like 1) Carcinoma, 2) Sarcoma, 3) Carcinosarcoma, 4) Cancers of the blood-forming tissues, 5) tumors of nerve tissues including the brain, 6) cancer of skin cells. Cancer according to 1) occurs in epithelial tissues, which cover the outer body (the skin) and line mucous membranes and the inner cavitary structures of organs e.g. such as the breast, lung, the respiratory and gastrointestinal tracts, the endocrine glands, and the genitourinary system. Ductal or glandular elements may persist in epithelial tumors, as in adenocarcinomas like e.g. thyroid adenocarcinoma, gastric adenocarcinoma, uterine adenocarcinoma. Cancers of the pavement-cell epithelium of the skin and of certain mucous membranes, such as e.g. cancers of the tongue, lip, larynx, urinary bladder, uterine cervix, or penis, may be termed epidermoid or squamous-cell carcinomas of the respective tissues and are in the scope of the definition of cancer as well. Cancer according to 2) develops in connective tissues, including fibrous tissues, adipose (fat) tissues, muscle, blood vessels, bone, and cartilage like e.g. osteogenic sarcoma; liposarcoma, fibrosarcoma, synovial sarcoma. Cancer according to 3) is cancer that develops in both epithelial and connective tissue. Cancer disease within the scope of this definition may be primary or secondary, whereby primary indicates that the cancer originated in the tissue where it is found rather than was established as a secondary site through metastasis from another lesion. Cancers and tumor diseases within the scope of this definition may be benign or malign and may affect all anatomical structures of the body of a mammal. By example but not limited to they comprise cancers and tumor diseases of I) the bone marrow and bone marrow derived cells (leukemias), II) the endocrine and exocrine glands like e.g. thyroid, parathyroid, pituitary, adrenal glands, salivary glands, pancreas III) the breast, like e.g. benign or malignant tumors in the mammary glands of either a male or a female, the mammary ducts, adenocarcinoma, medullary carcinoma, comedo carcinoma, Paget's disease of the nipple, inflammatory carcinoma of the young woman, IV) the lung, V) the stomach, VI) the liver and spleen, VII) the small intestine, VIII) the colon, IX) the bone and its supportive and connective tissues like malignant or benign bone tumour, e.g. malignant osteogenic sarcoma, benign osteoma, cartilage tumors; like malignant chondrosarcoma or benign chondroma; bone marrow tumors like malignant myeloma or benign eosinophilic granuloma, as well as metastatic tumors from bone tissues at other locations of the body; X) the mouth, throat, larynx, and the esophagus, XI) the urinary bladder and the internal and external organs and structures of the urogenital system of male and female like ovaries, uterus, cervix of the uterus, testes, and prostate gland, XII) the prostate, XIII) the pancreas, like ductal carcinoma of the pancreas; XIV) the lymphatic tissue like lymphomas and other tumors of lymphoid origin, XV) the skin, XVI) cancers and tumor diseases of all anatomical structures belonging to the respiration and respiratory systems including thoracal muscles and linings, XVII) primary or secondary cancer of the lymph nodes XVIII) the tongue and of the bony structures of the hard palate or sinuses, XVIV) the mouth, cheeks, neck and salivary glands, XX) the blood vessels including the heart and their linings, XXI) the smooth or skeletal muscles and their ligaments and linings, XXII) the peripheral, the autonomous, the central nervous system including the cerebellum, XXIII) the adipose tissue.

The human PDE4C is highly expressed in the following cancer tissues: stomach tumor, colon tumor, liver tumor, lung tumor. The expression in the above mentioned tissues and in particular the differential expression between diseased tissue stomach tumor and healthy tissue stomach demonstrates that the human PDE4C or mRNA can be utilized to diagnose of cancer. Additionally the activity of the human PDE4C can be modulated to treat cancer.

*Inflammatory Diseases*

Inflammatory diseases comprise diseases triggered by cellular or non-cellular mediators of the immune system or tissues causing the inflammation of body tissues and subsequently producing an acute or chronic inflammatory condition. Examples for such inflammatory diseases are hypersensitivity reactions of type I — IV, for example but not limited to hypersensitivity diseases of the lung including asthma, atopic diseases, allergic rhinitis or conjunctivitis, angioedema of the lids, hereditary angioedema, antireceptor hypersensitivity reactions and autoimmune diseases, Hashimoto's thyroiditis, systemic lupus erythematosus, Goodpasture's syndrome, pemphigus, myasthenia gravis, Grave's and Raynaud's disease, type B insulin-resistant diabetes, rheumatoid arthritis, psoriasis, Crohn's disease, scleroderma; mixed connective tissue disease, polymyositis, sarcoidosis, glomerulonephritis, acute or chronic host versus graft reactions.

The human PDE4C is highly expressed in the following tissues of the immune system and tissues responsive to components of the immune system as well as in the following tissues responsive to mediators of inflammation: ileum chronic inflammation, liver liver cirrhosis, spleen liver cirrhosis, lung COPD. The expression in the above mentioned tissues and in particular the differential expression between diseased tissue ileum chronic inflammation and healthy tissue ileum demonstrates that the human PDE4C or mRNA can be utilized to diagnose of inflammatory diseases. Additionally the activity of the human PDE4C can be modulated to treat inflammatory diseases.

*Disorders Related to Pulmology*

Asthma is thought to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to tissue destruction related to the disorder. Other resident cells, such as smooth muscle cells, lung epithelial cells, mucus-producing cells, and nerve cells may also be abnormal in individuals with asthma and may contribute to its pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic and disabling disorder requiring long-term management.

Chronic obstructive pulmonary (or airways) disease (COPD) is a condition defined physiologically as airflow obstruction that generally results from a mixture of emphysema and peripheral airway obstruction due to chronic bronchitis [Botstein, 1980]. Emphysema is characterised by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does also occur in non-smokers.

The human PDE4C is highly expressed in the following tissues of the respiratory system: fetal lung, lung right upper lobe, lung right mid lobe, lung right lower lobe, lung tumor, lung COPD. The expression in the above mentioned tissues and in particular the differential expression between diseased tissue lung tumor and healthy tissue lung demonstrates that the human PDE4C or mRNA can be utilized to diagnose of respiratory diseases. Additionally the activity of the human PDE4C can be modulated to treat those diseases.

*Applications*

The present invention provides for both prophylactic and therapeutic methods for disorders of the peripheral and central nervous system, cardiovascular diseases (in particular angina and coronary heart disease), hematological diseases, inflammation, cancer, gastroenterological diseases and respiratory disease.

The regulatory method of the invention involves contacting a cell with an agent that modulates one or more of the activities of PDE4C. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of PDE4C. Examples of such stimulatory agents include the active PDE4C and nucleic acid molecules encoding a portion of PDE4C. In another embodiment, the agent inhibits one or more of the biological activities of PDE4C. Examples of such inhibitory agents include antisense nucleic acid molecules and antibodies. These regulatory methods can be performed in vitro (e.g., by culturing the cell with the agent) or, alternatively, in vivo (e.g, by administering the agent to a subject). As such, the present invention provides methods of treating an individual afflicted with a disease or disorder characterized by unwanted expression or activity of PDE4C or a protein in the PDE4C signaling pathway. In one embodiment, the method involves administering an agent like any agent identified or being identifiable by a screening assay as described herein, or combination of such agents that modulate say upregulate or downregulate the expression or activity of PDE4C or of any protein in the PDE4C signaling pathway. In another embodiment, the method involves administering a regulator of PDE4C as therapy to compensate for reduced or undesirably low expression or activity of PDE4C or a protein in the PDE4C signaling pathway.

Stimulation of activity or expression of PDE4C is desirable in situations in which enzymatic activity or expression is abnormally low and in which increased activity is likely to have a beneficial effect. Conversely, inhibition of enzymatic activity or expression of PDE4C is desirable in situations in which activity or expression of PDE4C is abnormally high and in which decreasing its activity is likely to have a beneficial effect.

This invention is further illustrated by the following examples which should not be construed as limiting. The contents of all references, patents and published patent applications cited throughout this application are hereby incorporated by reference.

**Pharmaceutical Compositions**

This invention further pertains to novel agents identified by the above-described screening assays and uses thereof for treatments as described herein.

The nucleic acid molecules, polypeptides, and antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes pharmaceutical compositions comprising a regulator of PDE4C expression or activity (and/or a regulator of the activity or expression of a protein in the PDE4C signaling pathway) as well as methods for preparing such compositions by combining one or more such regulators and a pharmaceutically acceptable carrier. Also within the invention are pharmaceutical compositions comprising a regulator identified using the screening assays of the invention packaged with instructions for use. For regulators that are antagonists of PDE4C activity or which reduce PDE4C expression, the instructions would specify use of the pharmaceutical composition for treatment of hematological and cardiovascular diseases, disorders of the peripheral and central nervous system, COPD, asthma, genito-urological disorders and inflammation diseases. For regulators that are agonists of PDE4C activity or increase PDE4C expression, the instructions would specify use of the pharmaceutical composition for treatment of hematological and cardiovascular diseases, disorders of the peripheral and central nervous system, COPD, asthma, genito-urological disorders and inflammation diseases.

An inhibitor of PDE4C may be produced using methods which are generally known in the art. In particular, purified PDE4C may be used to produce antibodies or to screen libraries of pharmaceutical agents to identify those which specifically bind PDE4C. Antibodies to PDE4C may also be generated using methods that are well known in the art. Such antibodies may include, but are not limited to, polyclonal, monoclonal, chimeric, single chain antibodies, Fab fragments, and fragments produced by a Fab expression library. Neutralizing antibodies like those which inhibit dimer formation are especially preferred for therapeutic use.

In another embodiment of the invention, the polynucleotides encoding PDE4C, or any fragment or complement thereof, may be used for therapeutic purposes. In one aspect, the complement of the polynucleotide encoding PDE4C may be used in situations in which it would be desirable to block the transcription of the mRNA. In particular, cells may be transformed with sequences complementary to polynucleotides encoding PDE4C. Thus, complementary molecules or fragments may be used to modulate PDE4C activity, or to achieve regulation of gene function. Such technology is now well known in the art, and sense or antisense oligonucleotides or larger fragments can be designed from various locations along the coding or control regions of sequences encoding PDE4C.

Expression vectors derived from retroviruses, adenoviruses, or herpes or vaccinia viruses, or from various bacterial plasmids, may be used for delivery of nucleotide sequences to the targeted organ, tissue, or cell population. Methods which are well known to those skilled in the art can be used to construct vectors which will express nucleic acid sequence complementary to the polynucleotides of the gene encoding PDE4C. These techniques are described, for example, in [Scott and Smith (1990)].

Any of the therapeutic methods described above may be applied to any subject in need of such therapy, including, for example, mammals such as dogs, cats, cows, horses, rabbits, monkeys, and most preferably, humans.

An additional embodiment of the invention relates to the administration of a pharmaceutical composition containing PDE4C in conjunction with a pharmaceutically acceptable carrier, for any of the therapeutic effects discussed above. Such pharmaceutical compositions may consist of PDE4C, antibodies to PDE4C, and mimetics, agonists, antagonists, or inhibitors of PDE4C. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration. For pharmaceutical compositions which include an antagonist of PDE4C activity, a compound which reduces expression of PDE4C, or a compound which reduces expression or activity of a protein in the PDE4C signaling pathway or any combination thereof, the instructions for administration will specify use of the composition for hematological and cardiovascular diseases, disorders of the peripheral and central nervous system, COPD, asthma, genito-urological disorders and inflammation diseases. For pharmaceutical compositions which include an agonist of PDE4C activity, a compound which increases expression of PDE4C, or a compound which increases expression or activity of a protein in the PDE4C signaling pathway or any combination thereof, the instructions for administration will specify use of the composition for hematological and cardiovascular diseases, disorders of the peripheral and central nervous system, COPD, asthma, genito-urological disorders and inflammation diseases.

## Diagnostics

In another embodiment, antibodies which specifically bind PDE4C may be used for the diagnosis of disorders characterized by the expression of PDE4C, or in assays to monitor patients being treated with PDE4C or agonists, antagonists, and inhibitors of PDE4C. Antibodies useful for diagnostic purposes may be prepared in the same manner as those described above for therapeutics. Diagnostic assays for PDE4C include methods which utilize the antibody and a label to detect PDE4C in human body fluids or in extracts of cells or tissues. The antibodies may be used with or without modification, and may be labeled by covalent or non-covalent joining with a reporter molecule. A wide variety of reporter molecules, several of which are described above, are known in the art and may be used.

A variety of protocols for measuring PDE4C, including ELISAs, RIAs, and FACS, are known in the art and provide a basis for diagnosing altered or abnormal levels of PDE4C expression. Normal or standard values for PDE4C expression are established by combining body fluids or cell extracts taken from normal mammalian subjects, preferably human, with antibody to PDE4C under conditions suitable for complex formation. The amount of standard complex formation may be quantified by various methods, preferably by photometric means. Quantities of PDE4C expressed in subject samples from biopsied tissues are compared with the standard values. Deviation between standard and subject values establishes the parameters for diagnosing disease.

In another embodiment of the invention, the polynucleotides encoding PDE4C may be used for diagnostic purposes. The polynucleotides which may be used include oligonucleotide sequences, complementary RNA and DNA molecules, and PNAs. The polynucleotides may be used to detect and quantitate gene expression in biopsied tissues in which expression of PDE4C may be correlated with disease. The diagnostic assay may be used to distinguish between absence, presence, and excess expression of PDE4C, and to monitor regulation of PDE4C levels during therapeutic intervention.

Polynucleotide sequences encoding PDE4C may be used for the diagnosis of disorders of the peripheral and central nervous system, cardiovascular diseases (in particular angina and coronary heart disease), hematological diseases, inflammation, cancer, gastroenterological diseases and respiratory disease associated with expression of PDE4C. The polynucleotide sequences encoding PDE4C may be used in Southern, Northern, or dot-blot analysis, or other membrane-based technologies; in PCR technologies; in dipstick, pin, and ELISA assays; and in microarrays utilizing fluids or tissues from patient biopsies to detect altered PDE4C expression. Such qualitative or quantitative methods are well known in the art.

In a particular aspect, the nucleotide sequences encoding PDE4C may be useful in assays that detect the presence of associated disorders, particularly those mentioned above. The nucleotide sequences encoding PDE4C may be labelled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered from that of a comparable control sample, the nucleotide sequences have hybridized with nucleotide sequences in the sample, and the presence of altered levels of nucleotide sequences encoding PDE4C in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of disorders of the peripheral and central nervous system, cardiovascular diseases (in particular angina and coronary heart disease), hematological diseases, inflammation, cancer, gastroenterological diseases and respiratory disease associated with expression of PDE4C, a normal or standard profile for expression is established. This may be accomplished by combining body fluids or cell extracts taken from normal subjects, either animal or human, with a sequence, or a fragment thereof, encoding PDE4C, under conditions suitable for hybridization or amplification. Standard hybridization may be quantified by comparing the values obtained from normal subjects with values from an experiment in which a known amount of a substantially purified polynucleotide is used. Standard values obtained from normal samples may be compared with values obtained from samples from patients who are symptomatic for a disorder. Deviation from standard values is used to establish the presence of a disorder.

**Determination of a Therapeutically Effective Dose**

The determination of a therapeutically effective dose is well within the capability of those skilled in the art. A therapeutically effective dose refers to that amount of active ingredient which increases or decreases PDE4C activity relative to PDE4C activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of PDE4C gene or the activity of PDE4C by at least about 10, preferably about 50, more preferably about 75, 90, or 100% relative to the absence of the reagent. The effectiveness of the mechanism chosen to decrease the level of expression of PDE4C gene or the activity of PDE4C can be assessed using methods well known in the art, such as hybridization of nucleotide probes to PDE4C-specific mRNA, quantitative RT-PCR, immunologic detection of PDE4C, or measurement of PDE4C activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules of the invention are those nucleic acid molecules which are contained in a group of nucleic acid molecules consisting of (i) nucleic acid molecules encoding a polypeptide comprising the amino acid sequence of SEQ ID NO: 2, (ii) nucleic acid molecules comprising the sequence of SEQ ID NO: 1, (iii) nucleic acid molecules having the sequence of SEQ ID NO: 1, (iv)nucleic acid molecules the complementary strand of which hybridizes under stringent conditions to a nucleic acid molecule of (i), (ii), or (iii); and (v) nucleic acid molecules the sequence of which differs from the sequence of a nucleic acid molecule of (iii) due to the degeneracy of the genetic code, wherein the polypeptide encoded by said nucleic acid molecule has PDE4C activity.

Polypeptides of the invention are those polypeptides which are contained in a group of polypeptides consisting of (i) polypeptides having the sequence of SEQ ID NO: 2, (ii) polypeptides comprising the sequence of SEQ ID NO: 2, (iii) polypeptides encoded by nucleic acid molecules of the invention and (iv) polypeptides which show at least 99%, 98%, 95%, 90%, or 80% homology with a polypeptide of (i), (ii), or (iii), wherein said purified polypeptide has PDE4C activity.

An object of the invention is a method of screening for therapeutic agents useful in the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) contacting a test compound with a PDE4C polypeptide, (ii) detect binding of said test compound to said PDE4C polypeptide. E.g., compounds that bind to the PDE4C polypeptide are identified potential therapeutic agents for such a disease.

Another object of the invention is a method of screening for therapeutic agents useful in the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) determining the activity of a PDE4C polypeptide at a certain concentration of a test compound or in the absence of said test compound, (ii) determining the activity of said polypeptide at a different concentration of said test compound. E.g., compounds that lead to a difference in the activity of the PDE4C polypeptide in (i) and (ii) are identified potential therapeutic agents for such a disease.

Another object of the invention is a method of screening for therapeutic agents useful in the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) determining the activity of a PDE4C polypeptide at a certain concentration of a test compound, (ii) determining the activity of a PDE4C polypeptide at the presence of a compound known to be a regulator of a PDE4C polypeptide. E.g., compounds that show similar effects on the activity of the PDE4C polypeptide in (i) as compared to compounds used in (ii) are identified potential therapeutic agents for such a disease.

Other objects of the invention are methods of the above, wherein the step of contacting is in or at the surface of a cell.

Other objects of the invention are methods of the above, wherein the cell is in vitro.

Other objects of the invention are methods of the above, wherein the step of contacting is in a cell-free system.

Other objects of the invention are methods of the above, wherein the polypeptide is coupled to a detectable label.

Other objects of the invention are methods of the above, wherein the compound is coupled to a detectable label.

Other objects of the invention are methods of the above, wherein the test compound displaces a ligand which is first bound to the polypeptide.

Other objects of the invention are methods of the above, wherein the polypeptide is attached to a solid support.

Other objects of the invention are methods of the above, wherein the compound is attached to a solid support.

Another object of the invention is a method of screening for therapeutic agents useful in the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) contacting a test compound with a PDE4C polynucleotide, (ii) detect binding of said test compound to said PDE4C polynucleotide. Compounds that, e.g., bind to the PDE4C polynucleotide are potential therapeutic agents for the treatment of such diseases.

Another object of the invention is the method of the above, wherein the nucleic acid molecule is RNA.

Another object of the invention is a method of the above, wherein the contacting step is in or at the surface of a cell.

Another object of the invention is a method of the above, wherein the contacting step is in a cell-free system.

Another object of the invention is a method of the above, wherein the polynucleotide is coupled to a detectable label.

Another object of the invention is a method of the above, wherein the test compound is coupled to a detectable label.

Another object of the invention is a method of diagnosing a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) determining the amount of a PDE4C polynucleotide in a sample taken from said mammal, (ii) determining the amount of PDE4C polynucleotide in healthy and/or diseased mammal. A disease is diagnosed, e.g., if there is a substantial similarity in the amount of PDE4C polynucleotide in said test mammal as compared to a diseased mammal.

Another object of the invention is a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising a therapeutic agent which binds to a PDE4C polypeptide.

Another object of the invention is a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising a therapeutic agent which regulates the activity of a PDE4C polypeptide.

Another object of the invention is a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising a therapeutic agent which regulates the activity of a PDE4C polypeptide, wherein said therapeutic agent is (i) a small molecule, (ii) an RNA molecule, (iii) an antisense oligonucleotide, (iv) a polypeptide, (v) an antibody, or (vi) a ribozyme.

Another object of the invention is a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising a PDE4C polynucleotide.

Another object of the invention is a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising a PDE4C polypeptide.

Another object of the invention is the use of regulators of a PDE4C for the preparation of a pharmaceutical composition for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal.

Another object of the invention is a method for the preparation of a pharmaceutical composition useful for the treatment of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal comprising the steps of (i) identifying a regulator of PDE4C, (ii) determining whether said regulator ameliorates the symptoms of a disease comprised in a group of diseases consisting of disorders of the peripheral and central nervous system, cardiovascular diseases (in particular angina and coronary heart disease), hematological diseases, inflammation, cancer, gastroenterological diseases and respiratory disease in a mammal; and (iii) combining of said regulator with an acceptable pharmaceutical carrier.

Another object of the invention is the use of a regulator of PDE4C for the regulation of PDE4C activity in a mammal having a disease comprised in a group of diseases consisting of disorders of the peripheral and central nervous system, cardiovascular diseases (in particular angina and coronary heart disease), hematological diseases, inflammation, cancer, gastroenterological diseases and respiratory disease.

The examples below are provided to illustrate the subject invention. These examples are provided by way of illustration and are not included for the purpose of limiting the invention.

## Examples

### Example 1: Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For PDE4C the following hits to known sequences were identified by using the BLAST algorithm [Altschul SF, Madden TL, Schaffer AA, Zhang J, Zhang Z, Miller W, Lipman DJ; Nucleic Acids Res 1997 Sep 1; 25(17): 3389-402] and the following set of parameters: matrix = BLOSUM62 and low complexity filter. The following databases were searched: NCBI (non-redundant database) and DERWENT patent database (Geneseq).

The following hits were found:

>ref|NM_000923.1| Homo sapiens phosphodiesterase 4C, cAMP-specific (phosphodiesterase E1 dunce homolog, Drosophila) (PDE4C), mRNA, Length = 3495, Score = 6720 bits (3495), Expect = 0.0, Identities = 3495/3495 (100%) .

>emb|Z46632.1|HSPDE4C1 H.sapiens HSPDE4C1 gene for 3',5'-cyclic AMP phosphodiesterase, Length = 3495, Score = 6720 bits (3495), Expect = 0.0, Identities = 3495/3495 (100%)

>dbj|AK095384.1| Homo sapiens cDNA FLJ38065 fis, clone CTONG2015316, highly similar to CAMP-DEPENDENT 3',5'-CYCLIC PHOSPHODIESTERASE 4C (EC 3.1.4.17), Length = 3740, Score = 5495 bits (2858), Expect = 0.0, Identities = 2874/2882 (99%)

>gb|I73375.1|I73375 Sequence 1 from patent US 5686286, Length = 2091, Score = 3655 bits (1901), Expect = 0.0, Identities = 1915/1922 (99%)

>gb|I67721.1|I67721 Sequence 1 from patent US 5672509, Length = 2091, Score = 3655 bits (1901), Expect = 0.0, Identities = 1915/1922 (99%)

>emb|A52075.1|A52075 Sequence 31 from Patent WO9620281, Length = 2162, Score = 3617 bits (1881), Expect = 0.0, Identities = 1893/1899 (99%)

>gb|U88712.1|HSU88712 Human phosphodiesterase 4C mRNA, complete cds, Length = 2162, Score = 3617 bits (1881), Expect = 0.0, Identities = 1893/1899 (99%)

>gb|U88713.1|HSU88713 Human phosphodiesterase 4C mRNA, partial cds, Length = 2109, Score = 3615 bits (1880), Expect = 0.0, Identities = 1892/1899 (99%)

>gb|AR169829.1|AR169829 Sequence 31 from patent US 6291199, Length = 2153, Score = 3609 bits (1877), Expect = 0.0, Identities = 1889/1895 (99%)

>gb|AR067684.1|AR067684 Sequence 31 from patent US 5851784, Length = 2153, Score = 3609 bits (1877), Expect = 0.0, Identities = 1889/1895 (99%)

>gb|U66347.1|HSPDE4C2B Homo sapiens cAMP phosphodiesterase (PDE4C) mRNA, 4C-426 isoform, complete cds, Length = 3511, Score = 3269 bits (1700), Expect = 0.0, Identities = 1710/1715 (99%)

>gb|AC068499.1|AC068499 Homo sapiens chromosome 19, cosmid R26574 (LLNL-R_225F10), complete sequence, Length = 36431, Score = 3027 bits (1574), Expect = 0.0, Identities = 1589/1594 (99%), Gaps = 2/1594 (0%)

>gb|AF157816.1|HSPDE4CG7 Homo sapiens cAMP specific phosphodiesterase (PDE4C) gene, exons 14 and 15 and alternative splice products, complete cds, Length = 4734, Score = 2975 bits (1547), Expect = 0.0, Identities = 1584/1595 (99%), Gaps = 4/1595 (0%)

>gb|U66348.1|HSPDE4C2C Homo sapiens cAMP phosphodiesterase (PDE4C) mRNA, 4C-delta54 isoform, partial cds, Length = 1559, Score = 2728 bits (1419), Expect = 0.0, Identities = 1427/1431 (99%)

>gb|AC005759.1|AC005759 Homo sapiens chromosome 19, cosmid R26927, complete sequence, Length = 34688, Score = 2367 bits (1231), Expect = 0.0, Identities = 1233/1234 (99%)

>gb|I22499.1|I22499 Sequence 45 from patent US 5527896, Length = 1155, Score = 2196 bits (1142), Expect = 0.0, Identities = 1142/1142 (100%)

>gb|L20968.1|HUMPDED Human phosphodiesterase mRNA, 3' end, Length = 1155, Score = 2196 bits (1142), Expect = 0.0, Identities = 1142/1142 (100%)

>gb|AR083688.1|AR083688 Sequence 81 from patent US 5977305, Length = 934, Score = 1783 bits (927), Expect = 0.0, Identities = 927/927 (100%)

>gb|U66349.1|HSPDE4C2D Homo sapiens cAMP phosphodiesterase (PDE4C) mRNA, 4C-delta109 isoform, partial cds, Length = 1284, Score = 1740 bits (905), Expect = 0.0, Identities = 909/911 (99%)

>gb|M25347.1|RATPHOCN Rat cyclic nucleotide phosphodiesterase (PDE1) mRNA, partial cds, Length = 1077, Score = 1069 bits (556), Expect = 0.0, Identities = 844/988 (85%)

**Example 2: Expression profiling**

Total cellular RNA was isolated from cells by one of two standard methods: 1) guanidine isothiocyanate/Cesium chloride density gradient centrifugation [Kellogg, (1990)]; or with the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination.

For relative quantitation of the mRNA distribution of PDE4C, total RNA from each cell or tissue source was first reverse transcribed. 85 μg of total RNA was reverse transcribed using 1 μmole random hexamer primers, 0.5 mM each of dATP, dCTP, dGTP and dTTP (Qiagen, Hilden, Germany), 3000 U RnaseQut (Invitrogen, Groningen, Netherlands) in a final volume of 680 μl. The first strand synthesis buffer and Omniscript reverse transcriptase (2 u/μl) were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of PDE4C mRNA in cells and tissues the Perkin Elmer ABI Prism RTM. 7700 Sequence Detection system or Biorad iCycler was used according to the manufacturer's specifications and protocols. PCR reactions were set up to quantitate PDE4C and the housekeeping genes HPRT (hypoxanthine phosphoribosyltransferase), GAPDH (glyceraldehyde-3-phosphate dehydrogenase), β-actin, and others. Forward and reverse primers and probes for PDE4C were designed using the Perkin Elmer ABI Primer Express™ software and were synthesized by TibMolBiol (Berlin, Germany). The PDE4C forward primer sequence was: Primer1 (SEQ ID NO: 3). The PDE4C reverse primer sequence was Primer2 (SEQ ID NO: 5). Probe1 (SEQ ID NO: 4), labelled with FAM (carboxyfluorescein succinimidyl ester) as the reporter dye and TAMRA (carboxytetramethylrhodamine) as the quencher, is used as a probe for PDE4C. The following reagents were prepared in a total of 25 μl : 1x TaqMan buffer A, 5.5 mM $MgCl_2$, 200 nM of dATP, dCTP, dGTP, and dUTP, 0.025 U/μl AmpliTaq Gold™, 0.01 U/ μl AmpErase and Probe1 (SEQ ID NO: 4), PDE4C forward and reverse primers each at 200 nM, 200 nM PDE4C FAM/TAMRA-labelled probe, and 5 μl of template cDNA. Thermal cycling parameters were 2 min at 50°C, followed by 10 min at 95°C, followed by 40 cycles of melting at 95°C for 15 sec and annealing/extending at 60°C for 1 min.

For detection of PDE4A (Acc. No. P27815) mRNA, the following primers and probes were used: forward primer: SEQ ID NO: 6, reverse primer SEQ ID NO: 7, probe: SEQ ID NO: 8. The PDE4B (Acc. No. Q07343) forward primer was SEQ ID NO: 9, the PDE4B reverse primer was SEQ ID NO: 10, and the PDE4B probe was SEQ ID NO: 11. For detection of PDE4D (Acc. No. U79571) SEQ ID NO: 12 was used as a forward primer, SEQ ID NO: 13 as the reverse primer. The PDE4D probe was SEQ ID NO: 14. TaqMan conditions were the same as described for PDE4C.

*Calculation of corrected CT values*

The CT (threshold cycle) value is calculated as described in the "Quantitative determination of nucleic acids" section. The CF-value (factor for threshold cycle correction) is calculated as follows :

1. PCR reactions were set up to quantitate the housekeeping genes (HKG) for each cDNA sample.

2. $CT_{HKG}$-values (threshold cycle for housekeeping gene) were calculated as described in the "Quantitative determination of nucleic acids" section.

3. $CT_{HKG}$-mean values (CT mean value of all HKG tested on one cDNAs) of all HKG for each cDNA are calculated (n = number of HKG):

$$CT_{HKG\text{-}n}\text{-mean value} = (CT_{HKG1}\text{-value} + CT_{HKG2}\text{-value} + ... + CT_{HKG\text{-}n}\text{-value}) / n$$

4. $CT_{pannel}$ mean value (CT mean value of all HKG in all tested cDNAs) =

$$(CT_{HKG1}\text{-mean value} + CT_{HKG2}\text{-mean value} +...+ CT_{HKG-y}\text{-mean value}) / y$$

(y = number of cDNAs)

5. $CF_{cDNA-n}$ (correction factor for cDNA n) = $CT_{pannel}$-mean value - $CT_{HKG-n}$-mean value

6. $CT_{cDNA-n}$ (CT value of the tested gene for the cDNA n) + $CF_{cDNA-n}$ (correction factor for cDNA n) = $CT_{cor-cDNA-n}$ (corrected CT value for a gene on cDNA n)

*Calculation of relative expression*

Definition : highest $CT_{cor-cDNA-n} \neq 40$ is defined as $CT_{cor-cDNA}$ [high]

$$\text{Relative Expression} = 2^{(CTcor\text{-}cDNA[high]\ -\ CTcor\text{-}cDNA\text{-}n)}$$

*Tissues*

The expression of PDE4C was investigated in the tissues listed in tables 1-3.

*Expression profile*

The results of the the mRNA-quantification (expression profiling) is shown in Tables 1-3.

Table 1:

| *Relative expression of PDE4C in various human tissues* | |
|---|---|
| Tissue | Relative Expression |
| fetal heart | 223 |
| heart | 172 |
| pericardium | 352 |
| heart atrium (right) | 28 |
| heart atrium (left) | 37 |
| heart ventricle (left) | 30 |
| interventricular septum | 35 |
| fetal aorta | 1 |
| aorta | 8 |
| aorta sclerotic | 18 |
| artery | 24 |
| coronary artery | 21 |
| coronary artery sclerotic | 31 |
| vein | 27 |
| coronary artery smooth muscle primary cells | 63 |
| HUVEC cells | 87 |
| skin | 18 |
| adrenal gland | 8 |
| thyroid | 27 |
| thyroid tumor | 172 |
| pancreas | 15 |
| pancreas liver cirrhosis | 53 |

Table 1: (continued)

| *Relative expression of PDE4C in various human tissues* | |
|---|---|
| Tissue | Relative Expression |
| esophagus | 65 |
| esophagus tumor | 18 |
| stomach | 128 |
| stomach tumor | 50 |
| colon | 187 |
| colon tumor | 50 |
| small intestine | 57 |
| ileum | 91 |
| ileum tumor | 10 |
| ileum chronic inflammation | 588 |
| rectum | 407 |
| salivary gland | 1 |
| fetal liver | 16 |
| liver | 16 |
| liver liver cirrhosis | 410 |
| liver tumor | 17 |
| HEP G2 cells | 119 |
| leukocytes (peripheral blood) | 10 |
| Jurkat (T-cells) | 5 |
| bone marrow | 2 |
| erythrocytes | 27 |
| lymphnode | 44 |
| thymus | 3 |
| thrombocytes | 32 |
| bone marrow stromal cells | 2 |
| bone marrow CD71+ cells | 9 |
| bone marrow CD33+ cells | 32 |
| bone marrow CD34+ cells | 9 |
| bone marrow CD15+ cells | 24 |
| cord blood CD71+ cells | 9 |
| spleen | 139 |
| spleen liver cirrhosis | 495 |
| skeletal muscle | 174 |
| adipose | 55 |
| fetal brain | 171 |
| brain | 154 |
| Alzheimer brain | 122 |
| cerebellum | 1 |
| cerebellum (right) | 10 |
| cerebellum (left) | 17 |
| cerebral cortex | 115 |
| Alzheimer cerebral cortex | 114 |
| frontal lobe | 34 |
| Alzheimer brain frontal lobe | 51 |
| occipital lobe | 329 |

Table 1: (continued)

| *Relative expression of PDE4C in various human tissues* | |
|---|---|
| Tissue | Relative Expression |
| parietal lobe | 220 |
| temporal lobe | 302 |
| precentral gyrus | 195 |
| postcentral gyrus | 13 |
| tonsilla cerebelli | 11 |
| vermis cerebelli | 10 |
| pons | 67 |
| substantia nigra . | 110 |
| cerebral meninges | 22 |
| cerebral peduncles | 52 |
| corpus callosum | 22 |
| hippocampus | 357 |
| thalamus | 83 |
| dorsal root ganglia | 159 |
| spinal cord | 38 |
| neuroblastoma SK-N-MC cells | 12 |
| neuroblastoma SH-SY5Y cells | 74 |
| neuroblastoma IMR32 cells | 37 |
| glial tumor H4 cells | 22 |
| retina | 18 |
| | |
| fetal lung | 662 |
| fetal lung fibroblast IMR-90 cells | 9 |
| lung | 16 |
| lung right upper lobe | 40 |
| lung right mid lobe | 41 |
| lung right lower lobe | 42 |
| lung tumor | 350 |
| lung COPD | 28 |
| trachea | 26 |
| | |
| cervix | 12 |
| testis | 57 |
| HeLa cells (cervix tumor) | 14 |
| placenta | 40 |
| uterus | 78 |
| uterus tumor | 28 |
| ovary | 25 |
| ovary tumor | 98 |
| breast | 68 |
| breast tumor | 100 |
| MDA MB 231 cells (breast tumor) | 19 |
| mammary gland | 16 |
| | |
| prostate | 10 |
| prostate BPH | 17 |
| bladder | 20 |
| ureter | 95 |

Table 1: (continued)

| *Relative expression of PDE4C in various human tissues* | |
|---|---|
| Tissue | Relative Expression |
| penis | 46 |
| corpus cavernosum | 29 |
| fetal kidney | 146 |
| kidney | 119 |
| kidney tumor | 9 |
| HEK 293 cells | 58 |

Table 2

| *Relative expression of PDE4 subtypes in human myocardium and coronary arteries, each value represents the mean of 5 different specimen. Tissues examined were from different donors as those examined for table 1.* | | |
|---|---|---|
| PDE4 subtype | Expression (relative units) | |
| | Coronary artery | Myocardium |
| 4A | 5021 | 12297 |
| 4B | 1206 | 2663 |
| 4C | 816 | 206 |
| 4D | 236 | 1331 |

Table 3

| *Relative expression of PDE4C in human myocardium and coronary arteries, each value represents individual values of 2 different experiments. The difference in coronary artery expression between table 1 and table 3 can be explained by interindividual differences.* | | |
|---|---|---|
| Patient | Expression (relative units) | |
| | Coronary artery | Myocardium |
| 1 | 1459 | 139 |
| 2 | 488 | 315 |
| 3 | 402 | 410 |
| 4 | 1046 | 111 |
| 5 | 685 | 54 |

**Example 3: Animal Model "Anaesthetized Dog"**

The experiments were performed on dogs (mongrels) of either sex and a body weight between 20-30 kg. Induction of anaesthesia was achieved by slow iv injection of 25 mg/kg thiopental (Trapanal®). Anaesthesia was continued and maintained throughout the experiment by continuous infusion of 0.08 mg/kg/h fentanyl (fentanyl®) and 0.25 mg/kg/h droperidol (Dehydrobenzperidol®). Alloferin (0.02 mg/kg/h) was added as relaxant.
After intubation the dogs were artificially ventilated with one part $N_2O$ and 3 parts oxygen.
A tip catheter (PC 350, Millar Instruments) for recording left ventricular pressure was inserted into the ventricle via the carotid artery. Left ventricular contractility was calculated.
A hollow catheter was inserted into the femoral artery for recording of arterial blood pressure and connected to a strain gauge.
Two venous catheters were inserted into either femoral vein for drug and anaesthetic administration, respectively.
After a left-sided thoracotomy, the R. circumflexus (LCX) or the R. interventricularis (LAD) of the left coronary artery was freed from connective tissue and a electromagnetic flow probe (Gould Statham) was applied for measurement of coronary blood flow.
All primary signals were processed and stored electronically using the Ponemah software (Gould).

Simpson et al. investigated the effects of rolipram in anaesthetized dogs in an ischemia/reperfusion model [Simpson PJ et al., 1992]. Rolipram, using the high dose of 1 mg/kg iv + 1mg/kg/h infusion did not affect blood pressure and contractility.

We administered the same inhibitor (rolipram) using the very low doses (10-100 μg/kg/h) to anaesthetized dogs and investigated the cardiovascular effects. (A detailed description of the experimental is given in the attachment.) In contrast to the published data administration of rolipram at a dose of 30 μg/kg/h resulted in a slight increase in blood pressure, heart rate, contractility and coronary blood flow. These effects can be explained by an intracellular increase in cAMP in myocardial tissue; since the effects occur at very low doses it can be assumed that the effects are specific and most probably caused by PDE4 inhibition.

This finding is of particular relevance since the endogenous protective mechanisms (e.g. adenosine release under ischemic conditions) also act via an increase of cAMP. A low dose of a PDE4 inhibitor could act as a "drug on demand": Under normal conditions no cardiovascular effects will be seen. However, during ischemia (e.g. angina) the endogenous mechanisms which promote an increase of cAMP could be enhanced by inhibiting cAMP breakdown through PDE4 inhibition.

"Reactive hyperemia" can serve as a model for this pathophysiologic condition: in anaesthetized dogs (same experimental setup as described above) coronary blood flow is interrupted by short term ligation (4 min) of the coronary artery. Restoration of blood flow results in a reactive hyperemia: under control conditions blood flow increases by 106%, after 2.5 min the effect has subsided to 50%. During a low dose of rolipram which itself does not evoke cardiovascular effects (in this case 10 μg/kg/h) the observed reactive hyperemia is more pronounced (increase in flow +174%) and continues for a longer time ($T_{1/2}$ = 5 min).

A PDE4 inhibitor, which does not pass the blood-brain-barrier and/or has no emetic potential (achieved by its subtype selectivity), is a new principle in coronary therapy. Since PDE4C is preferentially expressed in coronary arteries, a specific PDE4C inhibitor is very well suited to increase coronary blood flow by simultaneously minimizing the effects on heart rate.

**Example 4: Antisense Analysis**

Knowledge of the correct, complete cDNA sequence coding for PDE4C enables its use as a tool for antisense technology in the investigation of gene function. Oligonucleotides, cDNA or genomic fragments comprising the antisense strand of a polynucleotide coding for PDE4C are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).

In addition to using sequences constructed to interrupt transcription of a particular open reading frame, modifications of gene expression is obtained by designing antisense sequences to intron regions, promoter/enhancer elements, or even to transacting regulatory genes.

**Example 5: Expression of PDE4C**

Expression of PDE4C is accomplished by subcloning the cDNAs into appropriate expression vectors and transfecting the vectors into expression hosts such as, e.g., *E. coli*. In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.

Induction of the isolated, transfected bacterial strain with Isopropyl-β-D-thiogalactopyranoside (IPTG) using standard methods produces a fusion protein corresponding to the first seven residues of β-galactosidase, about 15 residues of "linker", and the peptide encoded within the cDNA. Since cDNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.

The PDE4C cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.

Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E. coli*. For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.

Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced PDE4C are recovered from the conditioned medium and analyzed using chromatographic methods known in the art. For example, PDE4C can be cloned into the expression vector pcDNA3, as exemplified herein. This product can be used to transform, for example, HEK293 or COS by methodology standard in the art. Specifically, for example, using Lipofectamine (Gibco BRL catolog no. 18324-020) mediated gene transfer.

**Example 6: Isolation of Recombinant PDE4C**

PDE4C is expressed as a chimeric protein with one or more additional polypeptide domains added to facilitate protein purification. Such purification facilitating domains include, but are not limited to, metal chelating peptides such as histidine-tryptophan modules that allow purification on immobilized metals [Appa Rao, 1997] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the PDE4C sequence is useful to facilitate expression of PDE4C.

The following example provides a method for purifying PDE4C.

PDE4C is generated using the baculovirus expression system BAC-TO-BAC (GIBCO BRL) based on *Autographa californica* nuclear polyhedrosis virus (AcNPV) infection of *Spodoptera frugiperda* insect cells (Sf9 cells).

cDNA encoding PDE is cloned into either the donor plasmid pFASTBAC1 or pFASTBAC-HT which contain a mini-Tn7 transposition element. The recombinant plasmid is transformed into DH10BAC competent cells which contain the parent bacmid bMON14272 (AcNPV infectious DNA) and a helper plasmid. The mini-Tn7 element on the pFASTBAC donor can transpose to the attTn7 attachment site on the bacmid thus introducing the PDE gene into the viral genome. Colonies containing recombinant bacmids are identified by disruption of the *lac*Z gene. The PDE/bacmid construct can then be isolated and infected into insect cells (Sf9 cells) resulting in the production of infectious recombinant baculovirus particles and expression of either unfused recombinant enzyme (pFastbac1) or PDE4C-His fusion protein (pFastbacHT).

Cells are harvested and extracts prepared 24, 48 and 72 hours after transfection. Expression of PDE4C is confirmed by coomassie staining after sodium dodecyl sulphate-polyacrylamide gel electrophoresis (SDS-PAGE) and western blotting onto a PVDF membrane of an unstained SDS-PAGE. The PDE-His fusion protein is detected due to the interaction between the Ni-NTA HRP conjugate and the His-tag which is fused to PDE4C.

**Example 7: Production of PDE4C Specific Antibodies**

Two approaches are utilized to raise antibodies to PDE4C, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 μg are adequate for immunization of a mouse, while up to 1 mg might be used to immunize a rabbit. For identifying mouse hybridomas, the denatured protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate PDE4C domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431A using frnoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled PDE4C to identify those fusions producing the monoclonal antibody with the desired specificity. In a typical protocol, wells of plates (FAST; Becton-Dickinson, Palo Alto, CA) are coated during incubation with affinity purified, specific rabbit anti-mouse (or suitable antispecies 1 g) antibodies at 10 mg/ml. The coated wells are blocked with 1% bovine serum albumin, (BSA), washed and incubated with supernatants from hybridomas. After washing the wells are incubated with labeled PDE4C at 1 mg/ml. Supernatants with specific antibodies bind more labeled PDE4C than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least

$10^8$ $M^{-1}$, preferably $10^9$ to $10^{10}$ $M^{-1}$ or stronger, are typically made by standard procedures.

**Example 8: Diagnostic Test Using PDE4C Specific Antibodies**

Particular PDE4C antibodies are useful for investigating signal transduction and the diagnosis of infectious or hereditary conditions which are characterized by differences in the amount or distribution of PDE4C or downstream products of an active signaling cascade.

Diagnostic tests for PDE4C include methods utilizing antibody and a label to detect PDE4C in human body fluids, membranes, cells, tissues or extracts of such. The polypeptides and antibodies of the present invention are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound PDE4C, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on PDE4C is preferred, but a competitive binding assay may be employed.

**Example 9: Purification of Native PDE4C Using Specific Antibodies**

Native or recombinant PDE4C is purified by immunoaffinity chromatography using antibodies specific for PDE4C. In general, an immunoaffinity column is constructed by covalently coupling the anti-TRH antibody to an activated chromatographic resin.

Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of PDE4C by preparing a fraction from cells containing PDE4C in a soluble form. This preparation is derived by solubilization of whole cells or of a subcellular fraction obtained via differential centrifugation (with or without addition of detergent) or by other methods well known in the art. Alternatively, soluble PDE4C containing a signal sequence is secreted in useful quantity into the medium in which the cells are grown.

A soluble PDE4C-containing preparation is passed over the immunoaffinity column, and the column is washed under conditions that allow the preferential absorbance of PDE4C (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and PDE4C is collected.

**Example 10: Drug Screening**

This invention is particularly useful for screening therapeutic compounds by using PDE4C or fragments thereof in any of a variety of drug screening techniques.

The following example provides a system for drug screening measuring the phosphodiesterase activity.

The phosphodiesterase activity of the crude extracts is measured and confirmed that the PDE cDNA encodes a phosphodiesterase which is able to hydrolyze cAMP or cGMP or both.

The recombinant PDE-His fusion protein can be purified from the crude lysate by metal-affinity chromatography using Ni-NTA agarose. This allows the specific retention of the recombinant material (since this is fused to the His-tag) whilst the endogenous insect proteins are washed off. The recombinant material is then eluted by competition with imidazol.

The phosphodiesterase activity of the recombinant protein is assayed using a commercially available SPA (scintillation proximity assay) kit (Amersham Pharmacia). The PDE enzyme hydrolyzes cyclic nucleotides, e.g. cAMP and cGMP to their linear counterparts. The SPA assay utilizes the tritiated cyclic nucleotides [$^3$H]cAMP or [$^3$H]cGMP, and is based upon the selective interaction of the tritiated non cyclic product with the SPA beads whereas the cyclic substrates are not effectively binding. Radiolabelled product bound to the scintillation beads generates light that can be analyzed in a scintillation counter.

**Example 11: Rational Drug Design**

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the PDE4C amino acid sequence provided herein provides guidance to those employing computer modeling techniques in place of or in addition to x-ray crystallography.

**Example 12: Identification of Other Members of the Signal Transduction Complex**

Labeled PDE4C is useful as a reagent for the purification of molecules with which it interacts. In one embodiment of affinity purification, PDE4C is covalently coupled to a chromatography column. Cell-free extract derived from synovial cells or putative target cells is passed over the column, and molecules with appropriate affinity bind to PDE4C. PDE4C-complex is recovered from the column, and the PDE4C-binding ligand disassociated and subjected to N-terminal protein sequencing. The amino acid sequence information is then used to identify the captured molecule or to design degenerate oligonucleotide probes for cloning the relevant gene from an appropriate cDNA library.

In an alternate method, antibodies are raised against PDE4C, specifically monoclonal antibodies. The monoclonal antibodies are screened to identify those which inhibit the binding of labeled PDE4C. These monoclonal antibodies are then used therapeutically.

**Example 13: Use and Administration of Antibodies, Inhibitors, or Antagonists**

Antibodies, inhibitors, or antagonists of PDE4C or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to $10^5$ μg, up to a total dose of about 1 g, depending upon the route of administration. Guidance as to particular dosages and methods of delivery is provided in the literature; see U.S. Pat. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger PDE4C activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

**Example 14: Production of Non-human Transgenic Animals**

Animal model systems which elucidate the physiological and behavioral roles of the PDE4C are produced by creating nonhuman transgenic animals in which the activity of the PDE4C is either increased or decreased, or the amino acid sequence of the expressed PDE4C is altered, by a variety of techniques. Examples of these techniques include, but are not limited to: 1) Insertion of normal or mutant versions of DNA encoding a PDE4C, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these PDE4C sequences. The technique of homologous recombination is well known in the art. It replaces the native gene with the inserted gene and hence is useful for producing an animal that cannot express native PDE4Cs but does express, for example, an inserted mutant PDE4C, which has replaced the native PDE4C in the animal's genome by recombination, resulting in underexpression of the transporter. Microinjection adds genes to the genome, but does not remove them, and the technique is useful for producing an animal which expresses its own and added PDE4C, resulting in overexpression of the PDE4C.

One means available for producing a transgenic animal, with a mouse as an example, is as follows: Female mice are mated, and the resulting fertilized eggs are dissected out of their oviducts. The eggs are stored in an appropriate medium such as cesiumchloride M2 medium. DNA or cDNA encoding PDE4C is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit tissue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

## References

U.S. 4,522,811
U.S. 5,283,317
U.S. 5,527,896
U.S. 5,565,332
U.S. 5,672,509
U.S. 5,686,286
U.S. 5,851,784
U.S. 5,977,305
U.S. 6,291,199
WO 84/03564
WO 92/01810
WO 93/03151
WO 94/13804
WO 96/20281
WO 01/04297
Altschul SF, Madden TL, Schaffer AA, Zhang J, Zhang Z, Miller W, Lipman DJ; Nucleic Acids Res 1997 Sep 1; 25 (17): 3389-402
Appa Rao et al., 1997, Protein Expr Purif Nov, 11(2): 201-8
Bang, Y.J. et al. (1994) Proc. Natl. Acad. Sci. USA 91:5330-5334
Bardelle, C. et al. (1999) Anal. Biochem. 275: 148-155
Barnes, 2000, Chest, 117: 10S14S
Beavo, Physiol. Rev. 75:725-748 (1995);
Botstein et al., 1980, Am J Hum Genet. 32: 314-31
Charbonneau, et al., Proc. Natl. Acad. Sci . (USA) 83:9308-9312 (1986)
Colbere-Garapin *et al.*, 1981, *J. Mol. Biol. 150*, 1-14
Conti, et al., Physiol. Rev . 75:723-748 (1995)].
Conti et al. (1995) Endocrine Rev. 16:370-389
Dym O, Xenarios I, Ke H, Colicelli J; *Molec Pharmacol* 2002;61:20-25
Engelhard *et al.*, 1994, *Proc. Nat. Acad. Sci. 91,* 3224-3227
Francis, et al., J. Biol. Chem . 269:22477-22480 (1994).
Fujishige, K. et al. (1999) J. Biol. Chem. 274:18438-18445
Gergen and Weiss , 1992, Am Rev Respir Dis 146:823-824
Gibson et al., 1996, Genome Research 6: 995-1001
Han, P. et al. (1997) J. Biol. Chem. 272:16152-16157
Haseloff *et al.*, 1988 , *Nature 334*, 585-591
Harbinson, et al., Eur. Respir. J . 10:1008-1014 (1997)
Heid et al., 1996, Genome Research 6: 986-994
Hetman JM, Robas N, Baxendale R, Fidock M, Phillips SC, Soderling SH, Beavo JA Proc. Natl. Acad. Sci. U S A 2000 Nov 7;97(23):12891-5
Hansen, R.S., and Beavo, J.A., PNAS USA1982;79: 2788-92
Holland et al., 1991, PNAS 88: 7276-7280
Huang Z, Ducharme Y, Macdonald D, Robichaud A; *Current Opinion in Chemical Biology* 2001;5:432-438
Iwabuchi *et al.*, 1993, *Oncogene 8,* 1693-1696
Jeffreys et al., 1985, Nature 316: 76-9
Johnson *et al.*, 1989, *Endoc. Rev. 10*, 317-331
Kakkar, R. et al. (1999) Cell Mol Life Sci 55:1164-1186
Kellogg et al., 1990, Anal. Biochem. 189:202-208
Lam , 1997, Anticancer Drug Res. 12(3):145-67
Livak et al., 1995 , PCR Methods and Applications 357-362
Logan, Shenk, 1984, *Proc. Natl. Acad. Sci. 81*, 3655-3659
Loughney and Ferguson, in Phosphodiesterase Inhibitors, Schudt, et al. (Eds.), Academic Press: New York, N.Y. (1996) pp. 1- 19
Loughney, K. et al (1999) Gene 234:109-117
*Lowy et al.*, 1980, *Cell 22*, 817-23

Maddox *et al*., 1983, *J. Exp. Med. 158*, 1211-1216
McAllister-Lucas, L.M. (1995) J. Biol. Chem. 270:30671-30679
McConnell *et al.* , 1992 , *Science 257*, 1906-1912
Manganiello, et al., Arch. Biochem. Acta 322:1-13 (1995);
Nicholls *et al*., 1993, *J. Immunol. Meth. 165,* 81-91
Perry MJ et al., Cell Signal. 1997 Dec;9(8):575-85.
Perry, M.J. and G.A. Higgs (1998) Curr. Opin. Chem. Biol. 2:472-481
Piatak et al., 1993, BioTechniques 14:70-81
Piatak et al., 1993, Science 259:1749-1754
Porath *et al*., 1992, *Prot. Exp. Purif*. *3*, 263-281
Roberge *et al*., 1995, *Science 269,* 202-204
Sadhu, K. et al. (1999) J. Histochem Cytochem 47:895-906
Scott and Smith (1990) Science 249:386-390
Shelton et al., Biochim Biophys Acta. 1997 Sep 12;1353(3):287-97.
Simpson PJ, Schelm JA, Smallwood JK, Clay MP, Lindstrom TD; *J Cardiovasc Pharmacol* 1992;19:987-995
Sjolander, Urbaniczky, 1991, *Anal. Chem. 63*, 2338-2345
Soderling, S.H. et al. (1999) Proc. Natl. Acad. Sci. USA 96:7071-7076
Szabo *et al*., 1995, *Curr. Opin. Struct. Biol. 5*, 699-705
Terrett, N. et al. (1996) Bioorg. Med. Chem. Lett. 6:1819-1824
Thomas, 1980, Proc. Nat. Acad. Sci., 77:5201-5205
Turko, I.V. et al. (1998) Biochemistry 37:4200-4205
Ückert S, Küthe A, Stief CG, Jonas U; *World J Urol* 2001; 19:14-22
Uhlmann *et al*., 1987, *Tetrahedron. Lett. 215, 3539-3542*
Weber et al., 1990, Genomics 7: 524-30
Wigler *et al*., 1977, *Cell 11*, 223-32
Wigler *et al*., 1980, *Proc. Natl. Acad. Sci. 77*, 3567-70

## Claims

**1.** A method of screening for therapeutic agents useful in the treatment of a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) contacting a test compound with a PDE4C polypeptide,

ii) detect binding of said test compound to said PDE4C polypeptide.

**2.** A method of screening for therapeutic agents useful in the treatment of a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) determining the activity of a PDE4C polypeptide at a certain concentration of a test compound or in the absence of said test compound,

ii) determining the activity of said polypeptide at a different concentration of said test compound.

**3.** A method of screening for therapeutic agents useful in the treatment of a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) determining the activity of a PDE4C polypeptide at a certain concentration of a test compound,

ii) determining the activity of a PDE4C polypeptide at the presence of a compound known to be a regulator of a PDE4C polypeptide.

**4.** The method of any of the claims 1 to 3, wherein the cardiovascular disease is angina or coronary heart disease.

**5.** The method of any of claims 1 to 4, wherein the step of contacting is in or at the surface of a cell.

**6.** The method of any of claims 1 to 4, wherein the cell is in vitro.

**7.** The method of any of claims 1 to 4, wherein the step of contacting is in a cell-free system.

**8.** The method of any of claims 1 to 4, wherein the polypeptide is coupled to a detectable label.

**9.** The method of any of claims 1 to 4, wherein the compound is coupled to a detectable label.

**10.** The method of any of claims 1 to 4, wherein the test compound displaces a ligand which is first bound to the polypeptide.

**11.** The method of any of claims 1 to 4, wherein the polypeptide is attached to a solid support.

**12.** The method of any of claims 1 to 4, wherein the compound is attached to a solid support.

**13.** A method of screening for therapeutic agents useful in the treatment of a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) contacting a test compound with a PDE4C polynucleotide,

ii) detect binding of said test compound to said PDE4C polynucleotide.

**14.** The method of claim 13 wherein the nucleic acid molecule is RNA.

**15.** The method of claim 13 wherein the contacting step is in or at the surface of a cell.

**16.** The method of claim 13 wherein the contacting step is in a cell-free system.

**17.** The method of claim 13 wherein polynucleotide is coupled to a detectable label.

**18.** The method of claim 13 wherein the test compound is coupled to a detectable label.

**19.** The methods of claims 13 to 18, wherein the cardiovascular disease is angina or coronary heart disease.

**20.** A method of diagnosing a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) determining the amount of a PDE4C polynucleotide in a sample taken from said mammal,

ii) determining the amount of PDE4C polynucleotide in healthy and/or diseased mammals.

**21.** The method of claim 20, wherein the cardiovascular disease is angina or coronary heart disease.

**22.** A pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal comprising a therapeutic agent which binds to a PDE4C polypeptide.

**23.** The pharmaceutical composition of claim 22, wherein the cardiovascular disorder is angina or coronary heart disease.

**24.** A pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal comprising a therapeutic agent which regulates the activity of a PDE4C polypeptide.

**25.** The pharmaceutical composition of claim 24, wherein the cardiovascular disorder is angina or coronary heart disease.

**26.** A pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal comprising a therapeutic agent which regulates the activity of a PDE4C polypeptide, wherein said therapeutic agent is

i) a small molecule,
ii) an RNA molecule,
iii) an antisense oligonucleotide,
iv) a polypeptide,
v) an antibody, or
vi) a ribozyme.

**27.** The pharmaceutical composition of claim 26, wherein the cardiovascular disorder is angina or coronary heart disease.

**28.** A pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal comprising a PDE4C polynucleotide.

**29.** The pharmaceutical composition of claim 28, wherein the cardiovascular disorder is angina or coronary heart disease.

**30.** A pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal comprising a PDE4C polypeptide.

**31.** The pharmaceutical composition of claim 30, wherein the cardiovascular disorder is angina or coronary heart disease.

**32.** Use of regulators of a PDE4C for the preparation of a pharmaceutical composition for the treatment of a disease comprised in a group of diseases consisting of disorders of the peripheral and central nervous system, cardiovascular diseases, hematological diseases, inflammation, cancer, gastroenterological diseases and respiratory disease in a mammal.

**33.** The method of claim 32, wherein the cardiovascular disorder is angina or coronary heart disease.

**34.** Method for the preparation of a pharmaceutical composition useful for the treatment of a disease comprised in a group of diseases consisting of disorders of the peripheral and central nervous system, cardiovascular diseases, hematological diseases, inflammation, cancer, gastroenterological diseases and respiratory disease in a mammal comprising the steps of

i) identifying a regulator of PDE4C,

ii) determining whether said regulator ameliorates the symptoms of a disease comprised in a group of diseases consisting of disorders of the peripheral and central nervous system, cardiovascular diseases, hematological diseases, inflammation, cancer, gastroenterological diseases and respiratory disease in a mammal; and

iii) combining of said regulator with an acceptable pharmaceutical carrier.

**35.** The method of claim 34, wherein the cardiovascular disorder is angina or coronary heart disease.

**36.** Use of a regulator of PDE4C for the regulation of PDE4C activity in a mammal having a disease comprised in a group of diseases consisting of disorders of the peripheral and central nervous system, cardiovascular diseases, hematological diseases, inflammation, cancer, gastroenterological diseases and respiratory disease.

**37.** The method of claim 36, wherein the cardiovascular disorder is angina or coronary heart disease.

**Fig. 1**

**SEQ ID NO: 1**

CCAGTCTGCGGACCGTTCGGAGCAACGTGGCGGCCCTTGCCCGCCAGC
AATGCCTAGGAGCAGCCAAGCAGGGACCCGTCGGAAACCCTTCATCCA
GCCTTTCTCTGGCGCATGGAGAACCTGGGGGTCGGCGAAGGGGCAGAG
GCTTGCAGCAGGTTGAGTCGCTCTCGCGGCCGCCACAGCATGACCAGA
GCCCCGAAGCACCTGTGGCGGCAACCCCGGCGCCCCATCCGCATCCAA
CAGCGCTTCTATTCGGATCCGGACAAGTCCGCGGGCTGCCGCGAGAGG
GACCTGAGCCCGCGGCCGGAGCTCAGGAAGTCGCGGCTCTCCTGGCCC
GTTTCCTCCTGCAGGCGCTTTGACCTGGAAAATGGGCTCTCGTGTGGG
AGGAGGGCCCTGGACCCTCAGTCCAGCCCTGGCCTGGGCCGGATTATG
CAGGCTCCAGTCCCGCACAGCCAGCGGCGCGAGTCCTTCCTGTACCGC
TCAGATAGCGACTATGAACTCTCGCCCAAGGCCATGTCTCGGAACTCC
TCTGTGGCCAGCGACCTACATGGAGAGGACATGATTGTGACGCCCTTT
GCCCAGGTCCTGGCCAGTCTGCGGACCGTTCGGAGCAACGTGGCGGCC
CTTGCCCGCCAGCAATGCCTAGGAGCAGCCAAGCAGGGACCCGTCGGA
AACCCTTCATCCAGCAATCAGCTCCCTCCTGCAGAGGACACGGGGCAG
AAGCTGGCATTGGAGACGCTAGACGAGCTGGACTGGTGCCTGGATCAG
TTGGAGACGCTGCAGACCCGGCACTCGGTGGGGGAGATGGCCTCCAAC
AAGTTCAAGCGGATCCTGAACCGGGAGTTGACCCACCTGTCCGAAACC
AGCCGCTCCGGGAACCAGGTGTCCGAGTACATCTCCCGGACCTTCCTG
GACCAGCAGACCGAGGTGGAGCTGCCCAAGGTGACCGCTGAGGAGGCC
CCACAGCCCATGTCCCGGATCAGTGGCCTACATGGGCTCTGCCACAGT
GCCAGCCTCTCCTCAGCCACTGTCCCACGCTTTGGGGTCCAGACTGAC
CAGGAGGAGCAACTGGCCAAGGAGCTAGAAGACACCAACAAGTGGGGA
CTTGATGTGTTCAAGGTGGCGGACGTAAGTGGGAACCGGCCCCTCACA
GCTATCATATTCAGCATTTTTCAGGAGCGGGACCTGCTGAAGACATTC
CAGATCCCAGCAGACACACTGGCCACCTACCTGCTGATGCTGGAGGGT
CACTACCACGCCAATGTGGCCTACCACAACAGCCTACATGCCGCCGAC

Fig. 1 (continued)

GTGGCCCAGTCCACGCATGTGCTGCTGGCTACGCCCGCCCTCGAGGCT
GTGTTCACAGACTTGGAAATCCTGGCTGCCCTCTTTGCAAGCGCCATC
CACGACGTGGACCATCCTGGGGTCTCCAACCAGTTTCTGATTAACACC
AACTCAGACGTGGCGCTTATGTACAACGACGCCTCGGTGCTGGAGAAC
CATCACCTGGCTGTGGGCTTCAAGCTGCTGCAGGCAGAGAACTGCGAT
ATCTTCCAGAACCTCAGCGCCAAGCAGCGACTGAGTCTGCGCAGGATG
GTCATTGACATGGTGCTGGCCACAGACATGTCCAAACACATGAACCTC
CTGGCCGACCTCAAGACCATGGTGGAGACCAAGAAGGTGACAAGCCTC
GGTGTCCTCCTCCTGGACAACTATTCCGACCGAATCCAGGTCTTGCAG
AACCTGGTGCACTGTGCTGATCTGAGCAACCCCACCAAGCCGCTGCCC
CTGTACCGCCAGTGGACGGACCGCATCATGGCCGAGTTCTTCCAGCAG
GGAGACCGCGAGCGTGAGTCGGGCCTGGACATCAGTCCCATGTGTGAC
AAGCATACGGCCTCAGTGGAGAAGTCCCAGGTGGGTTTCATTGACTAC
ATTGCTCACCCACTGTGGGAGACTTGGGCTGACCTGGTCCACCCAGAT
GCACAGGACCTGCTGGACACGCTGGAGGACAATCGAGAGTGGTACCAG
AGCAAGATCCCCCGAAGTCCCTCAGACCTCACCAACCCCGAGCGGGAC
GGGCCTGACAGATTCCAGTTTGAACTGACTCTGGAGGAGGCAGAGGAA
GAGGATGAGGAGGAAGAAGAGGAGGGGGAAGAGACAGCTTTAGCCAAA
GAGGCCTTGGAGTTGCCTGACACTGAACTCCTGTCCCCTGAAGCCGGC
CCAGACCCTGGGGACTTACCCCTCGACAACCAGAGGACTTAGGGCCAG
CCCTGCGTGAACTGCAGGGGCAATGGATGGTAAAGCCCTTTGGCTCTT
GGCAGGCAGACTTTCCAGGAAGAGGCTCCATGTGGCTCCTGCTTCACT
TTCCCACCCATTTAGGGAGACAATCAAGCTCTTAGTTATAGGTGGCTC
CCAGGGTCTAATTGGAGGCACCTGGCTGGGGTCCACTCTGACCCTAGA
CTTGCCTAAAAGAGCTCTCTAAGGGGCAGCCTCTTACGATGCCCTGGT
GTCTTTCTCCTGGGCTTCTATCCCTGTGAGGAGAGGTGCTGTCTGCTG
GAGCCTCTAGTCCACCCTCTCCAGTGGTCACTCTTGAGTCACATCTGT

Fig. 1 (continued)

CACTTAATTATTTCCTTCTTTATCAAATATTTATTGCTCATCTACTTC
GGGCCAGCTTTCTGCCTCTGTAGTAGCCCTGCACAAAGGGTGGGGAGT
CAGGAGACCATCCCAAAGGCATCTCCCTGTCTTCCTCTACCAAGCGGC
TCTCTGCAAGAGCATGGAAATGTGAGTGGGGAAAATTTTCAGCACCAA
AGCTTCACTCATACCCAGTTTTGTTTCTGAAACTACGGTAGGGGGCAG
GAAGAGGAGCAGAAAAGAAGGGCTGGGCAAGGCATAGTGGCTTATGCC
TGTAATCCCGGTACTTTGGGAGGCTGAGGTGGGAGGACTGCTTAAGCT
CAGGAGTTTGAGACCAGCCTGGGCAACATAGCAAGACCCCCACCATCT
CTGAAAAAAAAAATTAGCCAGGCATGGTGGTGTGCACCTGAGAATCCC
AGCTACTCAGAAGGTTGAGACAAAGGGGATCGCTTGAGCCCAGGAGTT
GGAGGCTGAAGAGAGCTATGACTGCATCACTGCACTCCAGCCTGGGCA
ACACAGCAAGATCCTGTCTAAAAATAAAAAGAAAAGAGAAGGAAAGGA
AAGAGACGGGGCTCTGAGGCCGAGCACAGTGGCCCATGCCTATAATCC
CAGCACTTTGGGAGGCTGAGGCAGGTGGATCACCTGAGGTTAGGAGTT
CGAGACCAGCCTGGCCAACATGGTGAAACCCCATCTCTACTAAAAATA
CAAAAATTGGCTGGGCATGGTGGCGGGTGCCTGTAATCCCAGCTACTG
GGGAGGCTGAGGCAGGAGAATCACTTGAATTCAGGAGGTGGAGGTTGC
AGTGAGCCGACATCATGCCACTGCACTCCAGCCTGGGGCTGACAGAGC
AAGACACTGTCTCAAAAAGAAAAAAAAAAAAAAAAAAA

Fig. 2

**SEQ ID NO: 2**

MENLGVGEGAEACSRLSRSRGRHSMTRAPKHLWRQPRRPIRIQQRFYS
DPDKSAGCRERDLSPRPELRKSRLSWPVSSCRRFDLENGLSCGRRALD
PQSSPGLGRIMQAPVPHSQRRESFLYRSDSDYELSPKAMSRNSSVASD
LHGEDMIVTPFAQVLASLRTVRSNVAALARQQCLGAAKQGPVGNPSSS
NQLPPAEDTGQKLALETLDELDWCLDQLETLQTRHSVGEMASNKFKRI
LNRELTHLSETSRSGNQVSEYISRTFLDQQTEVELPKVTAEEAPQPMS
RISGLHGLCHSASLSSATVPRFGVQTDQEEQLAKELEDTNKWGLDVFK
VADVSGNRPLTAIIFSIFQERDLLKTFQIPADTLATYLLMLEGHYHAN
VAYHNSLHAADVAQSTHVLLATPALEAVFTDLEILAALFASAIHDVDH
PGVSNQFLINTNSDVALMYNDASVLENHHLAVGFKLLQAENCDIFQNL
SAKQRLSLRRMVIDMVLATDMSKHMNLLADLKTMVETKKVTSLGVLLL
DNYSDRIQVLQNLVHCADLSNPTKPLPLYRQWTDRIMAEFFQQGDRER
ESGLDISPMCDKHTASVEKSQVGFIDYIAHPLWETWADLVHPDAQDLL
DTLEDNREWYQSKIPRSPSDLTNPERDGPDRFQFELTLEEAEEEDEEE
EEEGEETALAKEALELPDTELLSPEAGPDPGDLPLDNQRT

Fig. 3

**SEQ ID NO: 3**

5'-TTGATGTGTTCAAGGTGGCG-3'

Fig. 4

**SEQ ID NO: 4**

5'-GCTCCTGAAAAATGCTGAATATGA-3'

**Fig. 5**

**SEQ ID NO: 5**

5'-TAAGTGGGAACCGGCCCCTCACA-3'

**Fig. 6**

**SEQ ID NO: 6**

5'-CAGGAGATCTTGGACACTTTGGA-3'

**Fig. 7**

**SEQ ID NO: 7**

5'-GCATCCAGAGCTTCCTCGAC-3'

**Fig. 8**

**SEQ ID NO: 8**

5'-AGCCCAAGAGGCATTGACTGCGC-3'

**Fig. 9**

**SEQ ID NO: 9**

5'-GAGGGACACAGCTATTTCAGCA-3'

**Fig. 10**

**SEQ ID NO: 10**

5'-TCAGTCTCTCCCAGGGAATCTC-3'

**Fig. 11**

**SEQ ID NO: 11**

5'-CACAAAGACGCTTTGTGTGATTGATCCAGAA-3'

**Fig. 12**

**SEQ ID NO: 12**

5'-GCCAGTGCAATACATGATGTAGATC-3'

**Fig. 13**

**SEQ ID NO: 13**

5'-TGTACATCAAGGCAAGTTCAGAGTT-3'

**Fig. 14**

**SEQ ID NO: 14**

5'-TCCTGGTGTGTCCAATCAATTTCTGATCAA-3'

European Patent Office

# PARTIAL EUROPEAN SEARCH REPORT

which under Rule 45 of the European Patent Convention shall be considered, for the purposes of subsequent proceedings, as the European search report

Application Number
EP 02 02 6971

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PERSANI LUCA ET AL: "Relevant cAMP-specific phosphodiesterase isoforms in human pituitary: Effect of Gsalpha mutations." JOURNAL OF CLINICAL ENDOCRINOLOGY & METABOLISM, vol. 86, no. 8, August 2001 (2001-08), pages 3795-3800, XP001151837 ISSN: 0021-972X * the whole document * * abstract * * figure 1 * --- -/-- | 20, 22-27, 32-37 | G01N33/68 C12Q1/44 C12Q1/68 |

TECHNICAL FIELDS SEARCHED (Int.Cl.7)

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 14 May 2003 | Tuynman, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 02 02 6971

Although claim(s) 20 and 21 are directed to a diagnostic method practised on the human/animal body (Article 52(4) EPC), the search has been carried out and based on the alleged effects of the compound/composition.Although claims 36 and 37 are directed to a method of treatment of the human/animal body (Article 52(4) EPC), the search has been carried out and based on the alleged effects of the compound/composition.

---

Claim(s) searched completely:
1-19,22-35

Claim(s) searched incompletely:
20,21,36,37

Reason for the limitation of the search (non-patentable invention(s)):

Article 52 (4) EPC - Diagnostic method practised on the human or animal body
Article 52 (4) EPC - Method for treatment of the human or animal body by therapy

---

Further limitation of the search

Claim(s) searched completely:
1-21

Claim(s) searched incompletely:
22-29,32,33,36,37

Claim(s) not searched:
30,31

Reason for the limitation of the search:

Present claims 22-33 and 36 and 37 relate to a pharmaceutical compositions or regulators defined by reference to a desirable characteristic or property, namely being regulators of PDE4C expression or activity.

The claims cover all pharmaceutical compositions or regulators having this characteristic or property, whereas the application provides support within the meaning of Article 84 EPC and/or disclosure within the meaning of Article 83 EPC for only a very limited number of such pharmaceutical compositions or regulators. In the present case, the claims so lack support, and the application so lacks disclosure, that a meaningful search over the whole of the claimed scope is impossible. Independent of the above reasoning, the claims also lack clarity (Article 84 EPC). An attempt is made to define the pharmaceutical compositions or regulators by reference to a result to be achieved. Again, this lack of clarity in the present case is such as to render a meaningful search over the whole

European Patent Office

**INCOMPLETE SEARCH SHEET C**

Application Number
EP 02 02 6971

of the claimed scope impossible. Consequently, the search has been carried out for those parts of the claims which appear to be clear, supported and disclosed, namely those parts relating to the pharmaceutical compositions or regulators comprising rolipram (page 100, lines 19-26). Antisense oligonucleotides of PDE4C (page 82, lines 16-25) have also been searched in spite of their lack of disclosure, giving the applicant the benefit of doubt. For other proposed agents such as RNA molecules, polypeptides, antibodies and ribozymes the lack of support and or disclosure in the sense of Article 84 and 83 EPC, respectively, is such that a search is not considered meaningful, and therefore these embodiments have not been searched.

European Patent Office

# PARTIAL EUROPEAN SEARCH REPORT

Application Number
EP 02 02 6971

| Category | Citation of document with indication, where appropriate, of relevant passages | Relevant to claim | CLASSIFICATION OF THE APPLICATION (Int.Cl.7) |
|---|---|---|---|
| X | OWENS RAYMOND J ET AL: "Molecular cloning and expression of a human phosphodiesterase 4C." CELLULAR SIGNALLING, vol. 9, no. 8, December 1997 (1997-12), pages 575-585, XP002240988 ISSN: 0898-6568 * the whole document * * abstract * * page 575, left-hand column, line 7 - line 17 * | 1-7,9, 13-19, 22-29, 32-37 | |
| Y | | 8,10-12 | |
| X | TRIFILIEFF ALEXANDRE ET AL: "Pharmacological profile of a novel phosphodiesterase 4 inhibitor, 4-(8-benzo(1,2,5)oxadiazol-5-yl-(1,7)naphthyridin-6-yl)-benzoic acid (NVP-ABE171), a 1,7-naphthyridine derivative, with anti-inflammatory activities." JOURNAL OF PHARMACOLOGY AND EXPERIMENTAL THERAPEUTICS, vol. 301, no. 1, April 2002 (2002-04), pages 241-248, XP002240989 April, 2002 ISSN: 0022-3565 * the whole document * | 2,4, 22-27, 32-37 | TECHNICAL FIELDS SEARCHED (Int.Cl.7) |
| Y | | 3,5-12 | |

-/--

EPO FORM 1503 03.82 (P04C10)

European Patent Office

# PARTIAL EUROPEAN SEARCH REPORT

Application Number
EP 02 02 6971

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | HERSPERGER R ET AL: "Pd-Catalysed Cross-Coupling Reactions for the Synthesis of 6,8-Disubstituted 1,7-Naphtyridines: A Novel Class of Potent and Selective Phosphodiesterase Type 4D Inhibitors" JOURNAL OF MEDICINAL CHEMISTRY, AMERICAN CHEMICAL SOCIETY. WASHINGTON, US, vol. 43, 2000, pages 675-682, XP002234191 ISSN: 0022-2623 * the whole document * | 2,4, 22-27, 32-37 | |
| Y | --- | 3,5-12 | |
| X | SULLIVAN MICHAEL ET AL: "Genomic organisation of the human cyclic AMP-specific phosphodiesterase PDE4C gene and its chromosomal localisation to 19p13.1, between RAB3A and JUND." CELLULAR SIGNALLING, vol. 11, no. 10, October 1999 (1999-10), pages 735-742, XP002240990 ISSN: 0898-6568 * the whole document * * page 741, left-hand column, line 45 - line 56 * ----- | 13-18 | TECHNICAL FIELDS SEARCHED (Int.Cl.7) |

EPO FORM 1503 03.82 (P04C10)

SEQUENCE LISTING

<110> Bayer AG, BHC

<120> Diagnostics and Therapeutics for Diseases Associated with Human Phosphodiesterase 4C (PDE4C)

<130> Le A 36 410

<160> 14

<170> PatentIn version 3.1

<210> 1

<211> 3495

<212> DNA

<213> Homo sapiens

<400> 1

```
ccagtctgcg gaccgttcgg agcaacgtgg cggcccttgc ccgccagcaa tgcctaggag      60
cagccaagca gggacccgtc ggaaaccctt catccagcct ttctctggcg catggagaac     120
ctgggggtcg gcgaaggggc agaggcttgc agcaggttga gtcgctctcg cggccgccac     180
agcatgacca gagccccgaa gcacctgtgg cggcaacccc ggcgccccat ccgcatccaa     240
cagcgcttct attcggatcc ggacaagtcc gcgggctgcc gcgagaggga cctgagcccg     300
cggccggagc tcaggaagtc gcggctctcc tggcccgttt cctcctgcag gcgctttgac     360
ctggaaaatg ggctctcgtg tgggaggagg gccctggacc ctcagtccag ccctggcctg     420
ggccggatta tgcaggctcc agtcccgcac agccagcggc gcgagtcctt cctgtaccgc     480
tcagatagcg actatgaact ctcgcccaag gccatgtctc ggaactcctc tgtggccagc     540
gacctacatg gagaggacat gattgtgacg ccctttgccc aggtcctggc cagtctgcgg     600
accgttcgga gcaacgtggc ggcccttgcc cgccagcaat gcctaggagc agccaagcag     660
ggacccgtcg gaaacccttc atccagcaat cagctccctc ctgcagagga cacggggcag     720
aagctggcat tggagacgct agacgagctg gactggtgcc tggatcagtt ggagacgctg     780
cagacccggc actcggtggg ggagatggcc tccaacaagt tcaagcggat cctgaaccgg     840
gagttgaccc acctgtccga aaccagccgc tccgggaacc aggtgtccga gtacatctcc     900
cggaccttcc tggaccagca gaccgaggtg gagctgccca aggtgaccgc tgaggaggcc     960
ccacagccca tgtcccggat cagtggccta catgggctct gccacagtgc cagcctctcc    1020
```

tcagccactg tcccacgctt tggggtccag actgaccagg aggagcaact ggccaaggag 1080
ctagaagaca ccaacaagtg gggacttgat gtgttcaagg tggcggacgt aagtgggaac 1140
cggcccctca cagctatcat attcagcatt tttcaggagc gggacctgct gaagacattc 1200
cagatcccag cagacacact ggccacctac ctgctgatgc tggagggtca ctaccacgcc 1260
aatgtggcct accacaacag cctacatgcc gccgacgtgg cccagtccac gcatgtgctg 1320
ctggctacgc ccgccctcga ggctgtgttc acagacttgg aaatcctggc tgccctcttt 1380
gcaagcgcca tccacgacgt ggaccatcct ggggtctcca accagtttct gattaacacc 1440
aactcagacg tggcgcttat gtacaacgac gcctcggtgc tggagaacca tcacctggct 1500
gtgggcttca agctgctgca ggcagagaac tgcgatatct tccagaacct cagcgccaag 1560
cagcgactga gtctgcgcag gatggtcatt gacatggtgc tggccacaga catgtccaaa 1620
cacatgaacc tcctggccga cctcaagacc atggtggaga ccaagaaggt gacaagcctc 1680
ggtgtcctcc tcctggacaa ctattccgac cgaatccagg tcttgcagaa cctggtgcac 1740
tgtgctgatc tgagcaaccc caccaagccg ctgcccctgt accgccagtg gacggaccgc 1800
atcatggccg agttcttcca gcagggagac cgcgagcgtg agtcgggcct ggacatcagt 1860
cccatgtgtg acaagcatac ggcctcagtg gagaagtccc aggtgggttt cattgactac 1920
attgctcacc cactgtggga gacttgggct gacctggtcc acccagatgc acaggacctg 1980
ctggacacgc tggaggacaa tcgagagtgg taccagagca agatcccccg aagtccctca 2040
gacctcacca accccgagcg ggacgggcct gacagattcc agtttgaact gactctggag 2100
gaggcagagg aagaggatga ggaggaagaa gaggaggggg aagagacagc tttagccaaa 2160
gaggccttgg agttgcctga cactgaactc ctgtcccctg aagccggccc agaccctggg 2220
gacttacccc tcgacaacca gaggacttag ggccagccct gcgtgaactg caggggcaat 2280
ggatggtaaa gccctttggc tcttggcagg cagactttcc aggaagaggc tccatgtggc 2340
tcctgcttca ctttcccacc catttaggga gacaatcaag ctcttagtta taggtggctc 2400
ccagggtcta attggaggca cctggctggg gtccactctg accctagact tgcctaaaag 2460
agctctctaa ggggcagcct cttacgatgc cctggtgtct ttctcctggg cttctatccc 2520
tgtgaggaga ggtgctgtct gctggagcct ctagtccacc ctctccagtg gtcactcttg 2580
agtcacatct gtcacttaat tatttccttc tttatcaaat atttattgct catctacttc 2640
gggccagctt tctgcctctg tagtagccct gcacaaaggg tggggagtca ggagaccatc 2700
ccaaaggcat ctccctgtct tcctctacca agcggctctc tgcaagagca tggaaatgtg 2760
agtggggaaa attttcagca ccaaagcttc actcataccc agttttgttt ctgaaactac 2820
ggtagggggc aggaagagga gcagaaaaga agggctgggc aaggcatagt ggcttatgcc 2880
tgtaatcccg gtactttggg aggctgaggt gggaggactg cttaagctca ggagtttgag 2940
accagcctgg gcaacatagc aagaccccca ccatctctga aaaaaaaaat tagccaggca 3000
tggtggtgtg cacctgagaa tcccagctac tcagaaggtt gagacaaagg ggatcgcttg 3060
agcccaggag ttggaggctg aagagagcta tgactgcatc actgcactcc agcctgggca 3120
acacagcaag atcctgtcta aaaataaaaa gaaaagagaa ggaaaggaaa gagacggggc 3180
tctgaggccg agcacagtgg cccatgccta taatcccagc actttgggag gctgaggcag 3240
gtggatcacc tgaggttagg agttcgagac cagcctggcc aacatggtga aaccccatct 3300
ctactaaaaa tacaaaaatt ggctgggcat ggtggcgggt gcctgtaatc ccagctactg 3360
gggaggctga ggcaggagaa tcacttgaat tcaggaggtg gaggttgcag tgagccgaca 3420
tcatgccact gcactccagc ctggggctga cagagcaaga cactgtctca aaaaagaaaa 3480
aaaaaaaaaa aaaaa 3495

<210> 2

<211> 712

<212> PRT

<213> Homo sapiens

<400> 2

```
Met Glu Asn Leu Gly Val Gly Glu Gly Ala Glu Ala Cys Ser Arg Leu
1               5                   10                  15
Ser Arg Ser Arg Gly Arg His Ser Met Thr Arg Ala Pro Lys His Leu
            20                  25                  30
Trp Arg Gln Pro Arg Arg Pro Ile Arg Ile Gln Gln Arg Phe Tyr Ser
        35                  40                  45
Asp Pro Asp Lys Ser Ala Gly Cys Arg Glu Arg Asp Leu Ser Pro Arg
    50                  55                  60
Pro Glu Leu Arg Lys Ser Arg Leu Ser Trp Pro Val Ser Ser Cys Arg
65                  70                  75                  80
Arg Phe Asp Leu Glu Asn Gly Leu Ser Cys Gly Arg Arg Ala Leu Asp
                85                  90                  95
Pro Gln Ser Ser Pro Gly Leu Gly Arg Ile Met Gln Ala Pro Val Pro
            100                 105                 110
His Ser Gln Arg Arg Glu Ser Phe Leu Tyr Arg Ser Asp Ser Asp Tyr
        115                 120                 125
Glu Leu Ser Pro Lys Ala Met Ser Arg Asn Ser Ser Val Ala Ser Asp
    130                 135                 140
Leu His Gly Glu Asp Met Ile Val Thr Pro Phe Ala Gln Val Leu Ala
145                 150                 155                 160
Ser Leu Arg Thr Val Arg Ser Asn Val Ala Ala Leu Ala Arg Gln Gln
                165                 170                 175
Cys Leu Gly Ala Ala Lys Gln Gly Pro Val Gly Asn Pro Ser Ser Ser
            180                 185                 190
Asn Gln Leu Pro Pro Ala Glu Asp Thr Gly Gln Lys Leu Ala Leu Glu
        195                 200                 205
Thr Leu Asp Glu Leu Asp Trp Cys Leu Asp Gln Leu Glu Thr Leu Gln
    210                 215                 220
Thr Arg His Ser Val Gly Glu Met Ala Ser Asn Lys Phe Lys Arg Ile
225                 230                 235                 240
Leu Asn Arg Glu Leu Thr His Leu Ser Glu Thr Ser Arg Ser Gly Asn
                245                 250                 255
Gln Val Ser Glu Tyr Ile Ser Arg Thr Phe Leu Asp Gln Gln Thr Glu
            260                 265                 270
```

```
Val Glu Leu Pro Lys Val Thr Ala Glu Glu Ala Pro Gln Pro Met Ser
        275                 280                 285
Arg Ile Ser Gly Leu His Gly Leu Cys His Ser Ala Ser Leu Ser Ser
    290                 295                 300
Ala Thr Val Pro Arg Phe Gly Val Gln Thr Asp Gln Glu Glu Gln Leu
305                 310                 315                 320
Ala Lys Glu Leu Glu Asp Thr Asn Lys Trp Gly Leu Asp Val Phe Lys
                325                 330                 335
Val Ala Asp Val Ser Gly Asn Arg Pro Leu Thr Ala Ile Ile Phe Ser
            340                 345                 350
Ile Phe Gln Glu Arg Asp Leu Leu Lys Thr Phe Gln Ile Pro Ala Asp
        355                 360                 365
Thr Leu Ala Thr Tyr Leu Leu Met Leu Glu Gly His Tyr His Ala Asn
    370                 375                 380
Val Ala Tyr His Asn Ser Leu His Ala Ala Asp Val Ala Gln Ser Thr
385                 390                 395                 400
His Val Leu Leu Ala Thr Pro Ala Leu Glu Ala Val Phe Thr Asp Leu
                405                 410                 415
Glu Ile Leu Ala Ala Leu Phe Ala Ser Ala Ile His Asp Val Asp His
            420                 425                 430
Pro Gly Val Ser Asn Gln Phe Leu Ile Asn Thr Asn Ser Asp Val Ala
        435                 440                 445
Leu Met Tyr Asn Asp Ala Ser Val Leu Glu Asn His His Leu Ala Val
    450                 455                 460
Gly Phe Lys Leu Leu Gln Ala Glu Asn Cys Asp Ile Phe Gln Asn Leu
465                 470                 475                 480
Ser Ala Lys Gln Arg Leu Ser Leu Arg Arg Met Val Ile Asp Met Val
                485                 490                 495
Leu Ala Thr Asp Met Ser Lys His Met Asn Leu Leu Ala Asp Leu Lys
            500                 505                 510
Thr Met Val Glu Thr Lys Lys Val Thr Ser Leu Gly Val Leu Leu Leu
        515                 520                 525
Asp Asn Tyr Ser Asp Arg Ile Gln Val Leu Gln Asn Leu Val His Cys
    530                 535                 540
Ala Asp Leu Ser Asn Pro Thr Lys Pro Leu Pro Leu Tyr Arg Gln Trp
545                 550                 555                 560
Thr Asp Arg Ile Met Ala Glu Phe Phe Gln Gln Gly Asp Arg Glu Arg
                565                 570                 575
Glu Ser Gly Leu Asp Ile Ser Pro Met Cys Asp Lys His Thr Ala Ser
            580                 585                 590
Val Glu Lys Ser Gln Val Gly Phe Ile Asp Tyr Ile Ala His Pro Leu
        595                 600                 605
Trp Glu Thr Trp Ala Asp Leu Val His Pro Asp Ala Gln Asp Leu Leu
    610                 615                 620
```

Asp Thr Leu Glu Asp Asn Arg Glu Trp Tyr Gln Ser Lys Ile Pro Arg
625 630 635 640
Ser Pro Ser Asp Leu Thr Asn Pro Glu Arg Asp Gly Pro Asp Arg Phe
645 650 655
Gln Phe Glu Leu Thr Leu Glu Glu Ala Glu Glu Glu Asp Glu Glu Glu
660 665 670
Glu Glu Glu Gly Glu Glu Thr Ala Leu Ala Lys Glu Ala Leu Glu Leu
675 680 685
Pro Asp Thr Glu Leu Leu Ser Pro Glu Ala Gly Pro Asp Pro Gly Asp
690 695 700
Leu Pro Leu Asp Asn Gln Arg Thr
705 710

<210> 3

<211> 20

<212> DNA

<213> Homo sapiens

<400> 3
ttgatgtgtt caaggtggcg 20

<210> 4

<211> 24

<212> DNA

<213> Homo sapiens

<400> 4
gctcctgaaa aatgctgaat atga 24

<210> 5

<211> 23

<212> DNA

<213> Homo sapiens

<400> 5
taagtgggaa ccggcccctc aca 23

<210> 6

<211> 23

<212> DNA

<213> Homo sapiens

<400> 6
caggagatct tggacacttt gga 23

<210> 7

<211> 20

<212> DNA

<213> Homo sapiens

<400> 7
gcatccagag cttcctcgac 20

<210> 8

<211> 23

<212> DNA

<213> Homo sapiens

<400> 8
agcccaagag gcattgactg cgc 23

<210> 9

<211> 22

<212> DNA

<213> Homo sapiens

<400> 9
gagggacaca gctatttcag ca 22

<210> 10

<211> 22

<212> DNA

<213> Homo sapiens

<400> 10
tcagtctctc ccagggaatc tc 22

<210> 11

<211> 31

<212> DNA

<213> Homo sapiens

<400> 11
cacaaagacg ctttgtgtga ttgatccaga a 31

<210> 12

<211> 25

<212> DNA

<213> Homo sapiens

<400> 12
gccagtgcaa tacatgatgt agatc 25

<210> 13

<211> 25

<212> DNA

<213> Homo sapiens

<400> 13
tgtacatcaa ggcaagttca gagtt 25

<210> 14

<211> 30

<212> DNA

<213> Homo sapiens

<400> 14
tcctggtgtg tccaatcaat ttctgatcaa 30